# EUROPEAN PATENT APPLICATION

(11) **EP 4 298 896 A1**
(43) Date of publication of application: **03.01.2024**
(21) Application number: 23180527.6
(22) Date of filing: 21.06.2023
(51) Int. Cl.: A01H 1/04, A01H 5/08, A01H 6/34

(54) **NOVEL QTLS CONFERRING RESISTANCE TO CUCUMBER MOSAIC VIRUS**

(30) Priority: 21.06.2022 US 202263354067 P
(71) Applicant: Seminis Vegetable Seeds, Inc., St. Louis MO 63167 (US)
(72) Inventor: CANTET, Melissa, St. Louis, MO, 63167 (US); MONNOT, Severine, St. Louis, MO, 63167 (US)
(74) Representative: Uexküll & Stolberg

(57) **Abstract**

Cucumber plants exhibiting resistance to *Cucumber Mosaic Virus* (CMV) are provided, together with methods of producing, identifying, or selecting plants or germplasm with a *Cucumber Mosaic Virus* resistance phenotype. Such methods include producing a cucumber plant exhibiting resistance to CMV, comprising introgressing genomic regions conferring disease resistance; or selecting a cucumber plant exhibiting resistance to CMV. Compositions, including polymorphic markers for detecting plants comprising introgressed disease resistance alleles, are further provided.

## Description

A sequence listing containing the file named "SEMB048USP1_ST25.txt" which is 26 kilobytes (measured in MS-Windows^{®}) and created on June 21, 2022, and comprises 35 sequences, is incorporated herein by reference in its entirety.

### FIELD OF THE INVENTION

The present invention relates to the field of plant breeding and more specifically to methods and compositions for producing cucumber plants exhibiting improved resistance to *Cucumber mosaic virus* (CMV).

### BACKGROUND

Symptoms of *Cucumber mosaic virus* (CMV) typically include stunting of entire plants, mosaic or mottling, ring spots on leaves and fruits, and a variety of growth distortions such as cupping, puckering and strapping of leaves as well as warts on fruits. In extreme situations, parts of an affected plant or even an entire plant may die from the disease. Cucumber is one of the five most cultivated vegetables worldwide. Thus, resistance to CMV is a particularly important trait for the production of cucumbers. Although some CMV resistance alleles have been identified, the mapping and introduction of sustainable resistance to viruses remains one of the main challenges of modern plant breeding, especially in vegetables. Moreover, the globalization of food supply chains favors the spread of new virus strains or species. Therefore, a continuing need exists in the art to identify new resistance alleles conferring increased resistance to CMV as well as more effective methods of introgressing those resistance alleles into commercial lines to provide new varieties with improved resistance to CMV infection.

### SUMMARY

In one aspect, provided herein is a method of producing a cucumber plant exhibiting resistance to *Cucumber mosaic virus* (CMV), comprising introgressing into a plant a *Cucumber mosaic virus* resistance allele within a chromosomal segment flanked in the genome of the plant by marker locus M1 (SEQ ID NO:11) and marker locus M2 (SEQ ID NO:20) on chromosome 2; or marker locus M3 (SEQ ID NO:1) and marker locus M4 (SEQ ID NO: 10) on chromosome 6; wherein the introgressed CMV resistance allele confers to the plant increased resistance to CMV compared to a plant not comprising the allele. In some embodiments, the introgressing comprises: a) optionally crossing a cucumber plant comprising the chromosomal segment with itself or with a second cucumber plant to produce one or more progeny plants; and b) selecting a progeny plant comprising the chromosomal segment. In further embodiments, a representative sample of seed comprising the chromosomal segment on chromosome 2 has been deposited under ATCC Accession No. PTA-122638. In some embodiments, a representative sample of seed comprising the chromosomal segment on chromosome 6 has been deposited under ATCC Accession No. PTA-122638. In other embodiments, the *Cucumber mosaic virus* resistance allele is further defined as (a) located within a chromosomal segment flanked in the genome of said plant by marker locus M24 (SEQ ID NO:32) and marker locus M25 (SEQ ID NO:33) on chromosome 2; or (b) located within a chromosomal segment flanked in the genome of said plant by marker locus M26 (SEQ ID NO:34) and marker locus M27 (SEQ ID NO:35) on chromosome 6. In still other embodiments, the chromosomal segment comprises a marker locus on chromosome 2 selected from the group consisting of marker locus M5 (SEQ ID NO:12), marker locus M6 (SEQ ID NO:13), marker locus M7 (SEQ ID NO:14), marker locus M8 (SEQ ID NO:15), marker locus M9 (SEQ ID NO:16), marker locus M10 (SEQ ID NO:17), marker locus M11 (SEQ ID NO:18), and marker locus M12 (SEQ ID NO:19). In some embodiments, the chromosomal segment comprises a marker locus on chromosome 6 selected from the group consisting of marker locus M13 (SEQ ID NO:2), marker locus M14 (SEQ ID NO:3), marker locus M15 (SEQ ID NO:4), marker locus M16 (SEQ ID NO:5), marker locus M17 (SEQ ID NO:6), marker locus M18 (SEQ ID NO:7), marker locus M19 (SEQ ID NO:8), and marker locus M20 (SEQ ID NO:9). In further embodiments, the method further comprises introgressing into the plant a CMV resistance allele within a chromosomal segment flanked in the genome of the plant by: marker locus M21 (SEQ ID NO:29) and marker locus M22 (SEQ ID NO:30) on chromosome 5; marker locus KT316424 and marker locus KT316425 on chromosome 5; or marker locus SSR9-56 and marker locus SSR11-177 on chromosome 6. In certain embodiments, the method comprises introgressing into the plant a CMV resistance allele within a chromosomal segment flanked in the genome of the plant by marker locus M1 (SEQ ID NO:11) and marker locus M2 (SEQ ID NO:20) on chromosome 2 and a CMV resistance allele within a chromosomal segment flanked in the genome of the plant by marker locus M3 (SEQ ID NO:1) and marker locus M4 (SEQ ID NO:10) on chromosome 6. Introgressing may comprise backcrossing, marker-assisted selection, or assaying for the CMV resistance.

In another aspect, cucumber plants are provided that are obtainable by a method disclosed herein, wherein the plants comprise the CMV resistance allele within a chromosomal segment flanked in the genome of the plant by marker locus M1 (SEQ ID NO:11) and marker locus M2 (SEQ ID NO:20) on chromosome 2, or the CMV resistance allele within a chromosomal segment flanked in the genome of the plant by marker locus M3 (SEQ ID NO:1) and marker locus M4 (SEQ ID NO:10) on chromosome 6, or wherein the plant further comprises a CMV resistance allele within a chromosomal segment flanked in the genome of the plant by marker locus M21 (SEQ ID NO:29) and marker locus M22 (SEQ ID NO:30) on chromosome 5; or marker locus KT316424 and marker locus KT316425 on chromosome 5, including any possible combinations thereof.

In yet another aspect, methods are provided for selecting a cucumber plant with increased resistance to *Cucumber mosaic virus* (CMV) comprising: (a) optionally crossing a cucumber plant comprising a CMV resistance allele with a second cucumber plant to produce a population of progeny plants; and (b) selecting a progeny plant comprising the CMV resistance allele; wherein selecting the progeny plant comprises detecting a marker locus within or genetically linked to a chromosomal segment flanked in the genome of the plant by: marker locus M1 (SEQ ID NO:11) and marker locus M2 (SEQ ID NO:20) on chromosome 2; or marker locus M3 (SEQ ID NO:1) and marker locus M4 (SEQ ID NO:10) on chromosome 6. In some embodiments, selecting said progeny plant is further defined as detecting a marker locus within or genetically linked to a chromosomal segment flanked in the genome of said plant by: (a) marker locus M24 (SEQ ID NO:32) and marker locus M25 (SEQ ID NO:33) on chromosome 2; or (b) marker locus M26 (SEQ ID NO:34) and marker locus M27 (SEQ ID NO:35) on chromosome 6. In other embodiments, selecting a progeny plant comprises detecting nucleic acids comprising marker locus M5 (SEQ ID NO:12), marker locus M6 (SEQ ID NO:13), marker locus M7 (SEQ ID NO:14), marker locus M8 (SEQ ID NO:15), marker locus M9 (SEQ ID NO:16), marker locus M10 (SEQ ID NO:17), marker locus M11 (SEQ ID NO:18), marker locus M12 (SEQ ID NO:19), marker locus M13 (SEQ ID NO:2), marker locus M14 (SEQ ID NO:3), marker locus M15 (SEQ ID NO:4), marker locus M16 (SEQ ID NO:5), marker locus M17 (SEQ ID NO:6), marker locus M18 (SEQ ID NO:7), marker locus M19 (SEQ ID NO:8), or marker locus M20 (SEQ ID NO:9). In still other embodiments, the progeny plant is an F₂-F₆ progeny plant or producing the progeny plant comprises backcrossing.

In a further aspect, methods are provided for selecting a cucumber plant exhibiting resistance to *Cucumber mosaic virus* (CMV), comprising: a) obtaining a population of progeny plants having a parent comprising resistance to CMV; b) screening the population with at least one nucleic acid marker to detect a polymorphism genetically linked to CMV resistance; and c) selecting from the population one or more progeny plants comprising a haplotype associated with CMV resistance, wherein the haplotype comprises a CMV resistance allele flanked in the genome of the plant by: marker locus M1 (SEQ ID NO: 11) and marker locus M2 (SEQ ID NO:20) on chromosome 2; or marker locus M3 (SEQ ID NO:1) and marker locus M4 (SEQ ID NO: 10) on chromosome 6. In some embodiments, selecting the progeny plant comprises: (a) detecting a marker locus within or genetically linked to a chromosomal segment flanked in the genome of the plant by marker locus M1 (SEQ ID NO: 11) and marker locus M2 (SEQ ID NO:20) on chromosome 2; (b) detecting a marker locus within or genetically linked to a chromosomal segment flanked in the genome of the plant by marker locus M3 (SEQ ID NO:1) and marker locus M4 (SEQ ID NO: 10) on chromosome 6; or (c) detecting at least one polymorphism at a locus selected from the group consisting of marker locus marker locus M5 (SEQ ID NO: 12), marker locus M6 (SEQ ID NO:13), marker locus M7 (SEQ ID NO:14), marker locus M8 (SEQ ID NO: 15), marker locus M9 (SEQ ID NO:16), marker locus M10 (SEQ ID NO: 17), marker locus M11 (SEQ ID NO: 18), marker locus M12 (SEQ ID NO: 19), marker locus M13 (SEQ ID NO:2), marker locus M14 (SEQ ID NO:3), marker locus M15 (SEQ ID NO:4), marker locus M16 (SEQ ID NO:5), marker locus M17 (SEQ ID NO:6), marker locus M18 (SEQ ID NO:7), marker locus M19 (SEQ ID NO:8), and marker locus M20 (SEQ ID NO:9). In other embodiments, the progeny plant is an F₂-F₆ progeny plant or producing the progeny plant comprises backcrossing. In further embodiments, the screening the population comprises PCR, single strand conformational polymorphism analysis, denaturing gradient gel electrophoresis, cleavage fragment length polymorphism analysis, TAQMAN assay, and/or DNA sequencing. In certain embodiments, the CMV resistance cucumber plant comprises a CMV resistance allele on chromosome 2, a representative sample of seed comprising the allele has been deposited under ATCC Accession No. PTA-122638. In other embodiments, the CMV resistance cucumber plant comprises a CMV resistance allele on chromosome 6, a representative sample of seed comprising the allele has been deposited under ATCC Accession No. PTA-122638.

In yet another aspect, methods are provided for selecting a cucumber plant exhibiting resistance to *Cucumber mosaic virus* (CMV), comprising: a) screening one or more plants with at least one nucleic acid marker to detect a polymorphism genetically linked to CMV resistance; and b) selecting one or more plants comprising a haplotype associated with CMV resistance, wherein the haplotype comprises a CMV resistance allele flanked in the genome of the plant by: marker locus M1 (SEQ ID NO:11) and marker locus M2 (SEQ ID NO:20) on chromosome 2; or marker locus M3 (SEQ ID NO:1) and marker locus M4 (SEQ ID NO:10) on chromosome 6. In some embodiments, the CMV resistance allele is further defined as: (a) located within a chromosomal segment flanked in the genome of said plant by marker locus M24 (SEQ ID NO:32) and marker locus M25 (SEQ ID NO:33) on chromosome 2; or (b) located within a chromosomal segment flanked in the genome of said plant by marker locus M26 (SEQ ID NO:34) and marker locus M27 (SEQ ID NO:35) on chromosome 6. In other embodiments, selecting one or more plants comprises: (a) detecting a marker locus within or genetically linked to a chromosomal segment flanked in the genome of the plant by marker locus M1 (SEQ ID NO:11) and marker locus M2 (SEQ ID NO:20) on chromosome 2; (b) detecting a marker locus within or genetically linked to a chromosomal segment flanked in the genome of the plant by marker locus M3 (SEQ ID NO:1) and marker locus M4 (SEQ ID NO:10) on chromosome 6; or (c) detecting at least one polymorphism at a locus selected from the group consisting of marker locus marker locus M5 (SEQ ID NO:12), marker locus M6 (SEQ ID NO:13), marker locus M7 (SEQ ID NO:14), marker locus M8 (SEQ ID NO:15), marker locus M9 (SEQ ID NO:16), marker locus M10 (SEQ ID NO:17), marker locus M11 (SEQ ID NO:18), marker locus M12 (SEQ ID NO:19), marker locus M13 (SEQ ID NO:2), marker locus M14 (SEQ ID NO:3), marker locus M15 (SEQ ID NO:4), marker locus M16 (SEQ ID NO:5), marker locus M17 (SEQ ID NO:6), marker locus M18 (SEQ ID NO:7), marker locus M19 (SEQ ID NO:8), and marker locus M20 (SEQ ID NO:9). In certain embodiments, screening one or more plants comprises PCR, single strand conformational polymorphism analysis, denaturing gradient gel electrophoresis, cleavage fragment length polymorphism analysis, TAQMAN assay, and/or DNA sequencing.

In yet a further aspect, methods are provided for identifying a cucumber plant comprising a *Cucumber mosaic virus* (CMV) resistance allele: (a) obtaining nucleic acids from at least a first cucumber plant; and (b) identifying in the nucleic acids the presence of at least a first genetic marker indicative of the presence of a chromosomal segment flanked in the genome of the plant by: marker locus M1 (SEQ ID NO: 11) and marker locus M2 (SEQ ID NO:20) on chromosome 2; or marker locus M3 (SEQ ID NO:1) and marker locus M4 (SEQ ID NO: 10) on chromosome 6 wherein the CMV resistance allele confers to the plant increased resistance to CMV compared to a plant not comprising the allele. In some embodiments, the identifying comprises detecting a marker genetically linked to: (a) marker locus M5 (SEQ ID NO:12), marker locus M6 (SEQ ID NO:13), marker locus M7 (SEQ ID NO: 14), marker locus M8 (SEQ ID NO: 15), marker locus M9 (SEQ ID NO: 16), marker locus M10 (SEQ ID NO: 17), marker locus M11 (SEQ ID NO: 18), and marker locus M12 (SEQ ID NO:19); or (b) marker locus M13 (SEQ ID NO:2), marker locus M14 (SEQ ID NO:3), marker locus M15 (SEQ ID NO:4), marker locus M16 (SEQ ID NO:5), marker locus M17 (SEQ ID NO:6), marker locus M18 (SEQ ID NO:7), marker locus M19 (SEQ ID NO:8), and marker locus M20 (SEQ ID NO:9).

### BRIEF DESCRIPTION OF THE DRAWINGS

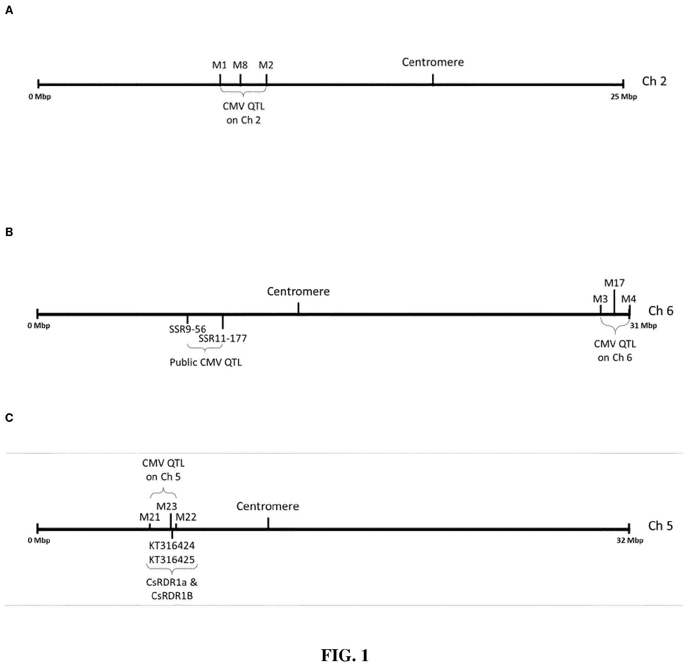

**FIG. 1****:** Shows a schematic overview of different *Cucumber mosaic virus* QTLs on the physical map of chromosomes 2 (Panel A), chromosome 6 (Panel B), and chromosome 5 (Panel C).

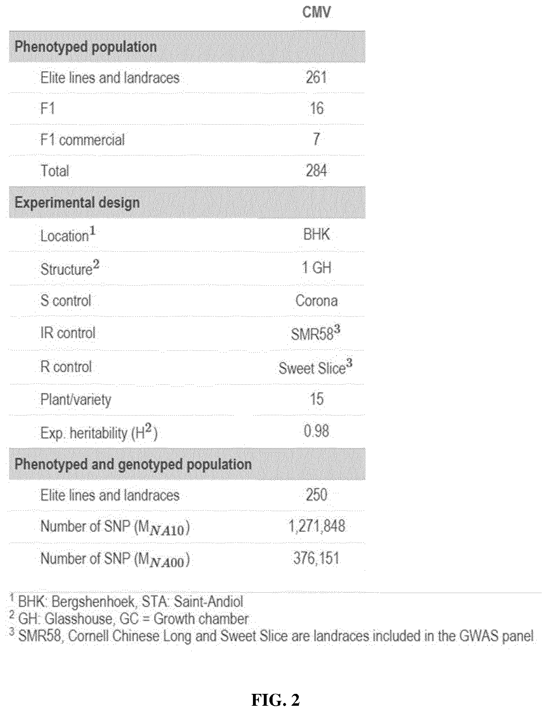

**FIG. 2****:** Shows a summary of the diversity panel used for the Genome Wide Association Studies (GWAS) including experimental design and heritability, population composition, and genomic variant number filtered for GWAS.

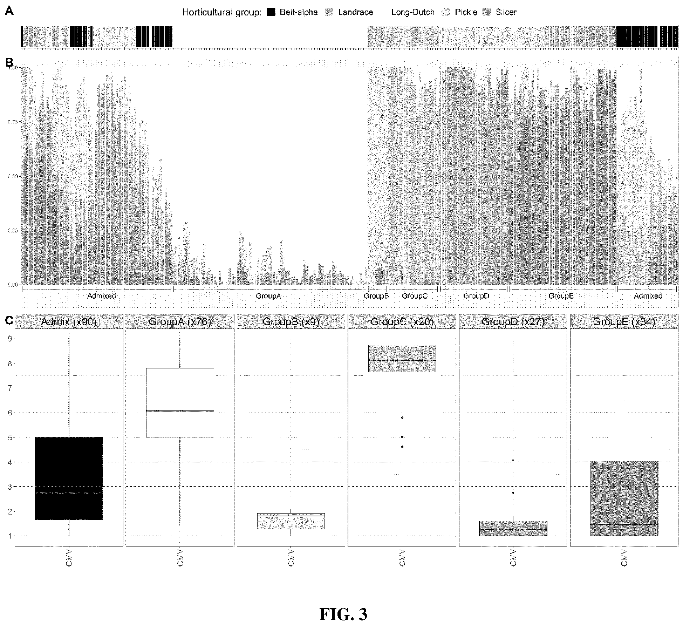

**FIG. 3****:** Shows the organization of cucumber accessions across five genetic groups (A) Each color represented the horticultural group (when known) of the accession. (B) Barplot representing the percentage attribution of cucumber accessions to nine genetic groups. Accessions were rarely attributed at 100% to one genetic group and the admixture observed results from crosses between breeding programs. Values were extracted with the snmf R package. Genetic groups were consistent with horticultural groups. (C) Boxplot representing the phenotypic variance in the diversity panel. Accessions were assigned to a genetic group when the contribution of this group was above 0.6, if no group reached this threshold, the accession was considered as admixed (in black). The number of accessions per group is indicated in brackets.

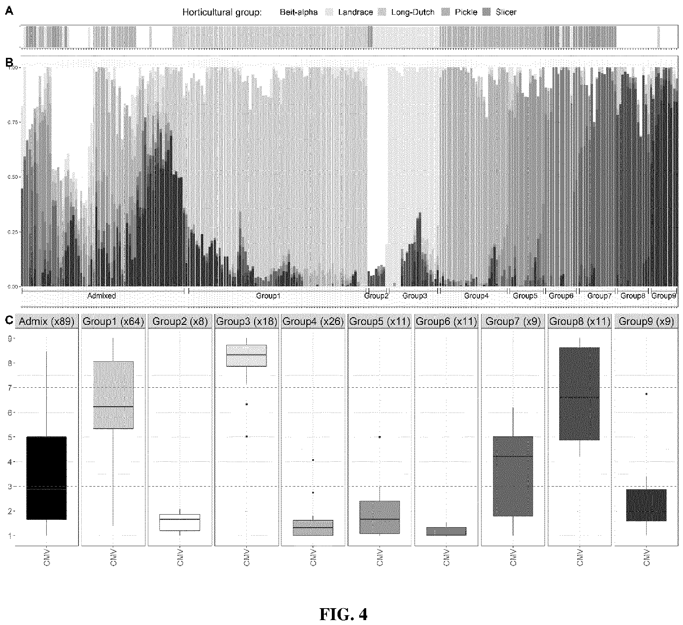

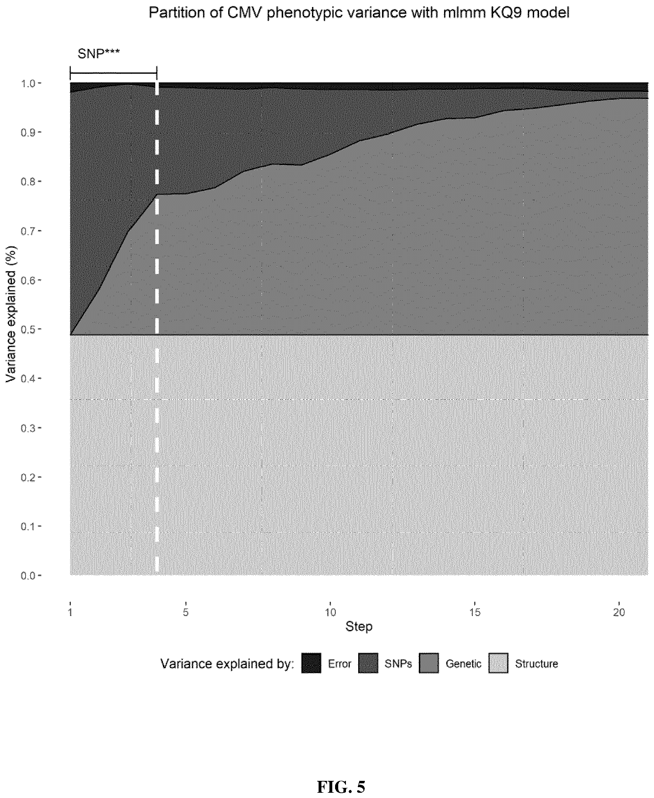

**FIG. 4****:** Shows the organization of cucumber accessions across nine genetic groups (A) Each color represented the horticultural group (when known) of the accession. (B) Barplot representing the percentage attribution of cucumber accessions to nine genetic groups. Accessions were rarely attributed at 100% to one genetic group and the admixture observed results from crosses between breeding programs. Values were extracted with the snmf R package. Genetic groups were consistent with horticultural groups. (C) Boxplot representing the phenotypic variance in the diversity panel for each virus. Accessions were assigned to a genetic group when the contribution of this group was above 0.6, if no group reached this threshold, the accession was considered as admixed (in black). The number of accessions per group is indicated in brackets. **FIG. 5****:** Shows the repartition of the phenotypic variance among (i) fixed effects either the genetic structure (lightest grey shade) and top SNPs from previous MLMM steps (middle grey shade) (ii) random effect (genetic, darkest grey shade) and (iii) error (black). The white dashed line indicates the significant SNPs using Bonferroni correction.

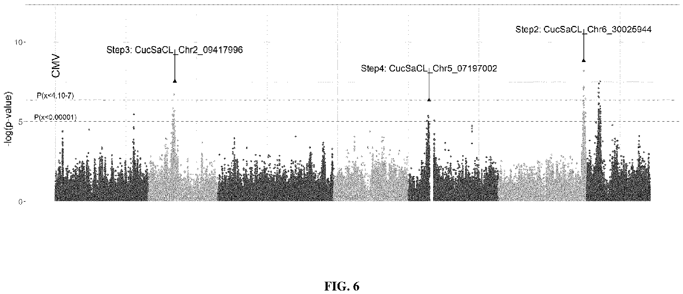

**FIG. 6****:** Shows a Manhattan plot corresponding to the GWAS analysis. The significance threshold is represented by the two dashed lines. The plots were built from p-values collected from the first KQ9 MLMM step (without marker correction). CMV had three isolated QTLs in chromosome 2, 5, and 6. The peak in chr7 disappeared as being redundant with the peak in chr6.

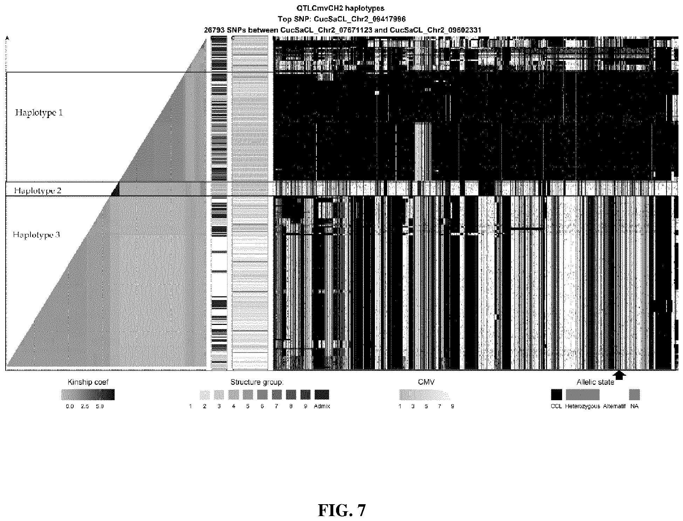

**FIG. 7****:** Shows the genetic organization of the QTL on chromosome 2 (26793 SNPs between 7.7 Mb and 9.6 Mb) in relation with genetic structure and level of resistance (A) Heatmap of the local kinship (B) Genetic group of accessions (cf color code Figure 4). (C) Resistance level to CMV 1-resistant, 9-susceptible. (D) Haplotype of each accession. Black when the SNP is homozygous as the reference genome CCL, white for alternative homozygous, grey for heterozygous. The black arrow (bottom) represents the location of the top SNP.

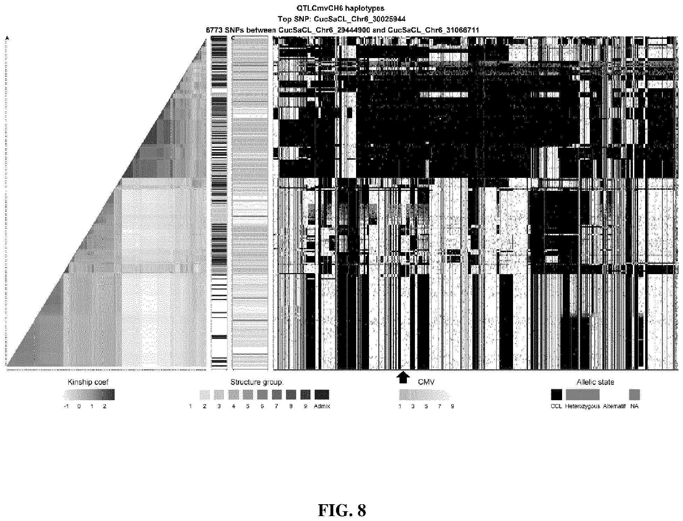

**FIG. 8****:** Shows the genetic organization of the QTL on chromosome 6 in relation with genetic structure and level of resistance (A) Heatmap of the local kinship (B) Genetic group of accessions (cf color code Figure 4). (C) Resistance level to CMV 1-resistant, 9-susceptible. (D) Haplotype of each accession. Black when the SNP is homozygous as the reference genome CCL, white for alternative homozygous, grey for heterozygous. The black arrow (bottom) represents the location of the top SNP.

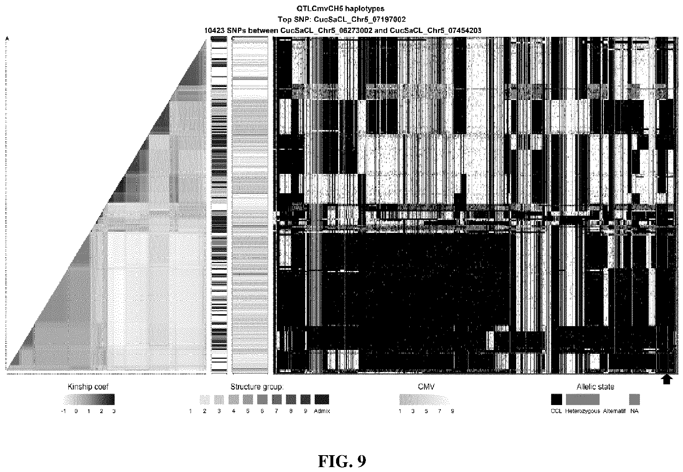

**FIG. 9****:** Shows the genetic organization of the QTL on chromosome 5 in relation with genetic structure and level of resistance (A) Heatmap of the local kinship (B) Genetic group of accessions (cf color code Figure 4). (C) Resistance level to CMV 1-resistant, 9-susceptible. (D) Haplotype of each accession. Black when the SNP is homozygous as the reference genome CCL, white for alternative homozygous, grey for heterozygous. The black arrow (bottom) represents the location of the top SNP.

### DETAILED DESCRIPTION

*Cucumber mosaic virus* (CMV) has one of the broadest host ranges of any virus throughout the temperate regions of the world. More than 800 species of plant can be infected, and more than 60 aphid species are vectors for this virus. CMV is a member of the cucumovirus group and is highly diverse. More than 60 strains of CMV have been identified.

Previously, one resistance QTL, identified as cmv6.1 and flanked by markers SSR9-56 and SSR11-177, has been mapped to chromosome 6 (Shi *et al.* 2018). Additionally, increased expression of a small gene family of RNA-dependent RNA polymerase 1 (RDR1) genes located on chromosome 5 in 'Shimshon', associated with marker locus KT316424 and marker locus KT316425, has been shown to be associated with increased resistance to CMV (Leibman *et al* 2018). However, the genetic architecture of cucumber resistance to CMV is complex and reports on resistance to CMV in cucumber are variable (Martín-Hernández et Picó 2021). Moreover, previous genetic studies appear to have only considered a tiny portion of the cucumber diversity to date.

In contrast, the present disclosure provides genome wide association studies (GWAS) using a large diversity panel in order to assess the architecture of cucumber resistance to CMV as a whole. Through an iterative GWAS (MLMM) approach that introduced as fixed effect the most significant SNPs from previous steps, three loci conferring resistance to CMV were identified on chromosome 2 ("chr2"), chromosome 5 ("chr5"), and chromosome 6 ("chr6"). Regarding the locus identified on chromosome 6, it was surprisingly found to be physically located more than 20 Mb away from cmv6.1. Thus, the loci on chromosome 2 and 6 described herein represent newly identified CMV resistance loci and provide valuable new tools for engineering CMV resistance in cucumber.

As disclosed herein, M1, a SNP marker with a [T/C] change at 7,671,123 bp on chromosome 2 of the public cucumber genome map version Cucumber Chinese Long' (CCL) landrace (Li *et al.* 2019), M2, a SNP marker with a [G/A] change at 9,602,331 bp on chromosome 2 of the public cucumber genome map version Cucumber Chinese Long' (CCL) landrace, M3, a SNP marker with a [A/T] change at 29,444,900 bp on chromosome 6 of the public cucumber genome map version Cucumber Chinese Long' (CCL) landrace, and M4, a SNP marker with a [C/A] change at 31,058,514 bp on chromosome 6 of the public cucumber genome map version Cucumber Chinese Long' (CCL) landrace, M21, a SNP marker with a [C/T] change at 6,284,008 bp on chromosome 5 of the public cucumber genome map version Cucumber Chinese Long' (CCL) landrace, and M22, a SNP marker with a [T/C] change at 7,437,769 bp on chromosome 5 of the public cucumber genome map version Cucumber Chinese Long' (CCL) landrace can be used to identify this region, wherein M1 and M2 are flanking markers for the chromosomal segment on chromosome 2; wherein M3 and M4 are flanking markers for the chromosomal segment on chromosome 6; and wherein M21 and M22 are flanking markers for the chromosomal segment on chromosome 5. The public genome of cucumber is available at for example Cucumber (Chinese Long) genome v3 at CuGenDB (http://cucurbitgenomics.org/) or at the Genbank assembly accession GCA_000004075.3 from NCBI (https://www.ncbi.nlm.nih.gov/genome/1639?genome_assembly_id=749658), and one skilled in the art would understand that the marker sequences provided for the first time in the instant application could be located on any version (or later version) of the public genome. One aspect of the present disclosure therefore provides methods of producing a cucumber plant exhibiting resistance to *Cucumber mosaic virus* (CMV), comprising introgressing into a plant a *Cucumber mosaic virus* resistance allele within a chromosomal segment flanked in the genome of the plant by marker locus M1 (SEQ ID NO:11) and marker locus M2 (SEQ ID NO:20) on chromosome 2; or marker locus M3 (SEQ ID NO:1) and marker locus M4 (SEQ ID NO:10) on chromosome 6; wherein the introgressed CMV resistance allele confers to the plant increased resistance to CMV compared to a plant not comprising the allele. In other aspects, the *Cucumber mosaic virus* resistance allele is further defined as (a) located within a chromosomal segment flanked in the genome of said plant by marker locus M24 (SEQ ID NO:32) and marker locus M25 (SEQ ID NO:33) on chromosome 2; or (b) located within a chromosomal segment flanked in the genome of said plant by marker locus M26 (SEQ ID NO:34) and marker locus M27 (SEQ ID NO:35) on chromosome 6.

In another aspect, cucumber plants are provided that are obtainable by a method disclosed herein, wherein the plants comprise the CMV resistance allele within a chromosomal segment flanked in the genome of the plant by marker locus M1 (SEQ ID NO:11) and marker locus M2 (SEQ ID NO:20) on chromosome 2, or the CMV resistance allele within a chromosomal segment flanked in the genome of the plant by marker locus M3 (SEQ ID NO:1) and marker locus M4 (SEQ ID NO:10) on chromosome 6, and wherein the plant further comprises a CMV resistance allele within a chromosomal segment flanked in the genome of the plant by marker locus M21 (SEQ ID NO:29) and marker locus M22 (SEQ ID NO:30) on chromosome 5; or marker locus KT316424 and marker locus KT316425 on chromosome 5, including any possible combinations thereof. In particular embodiments, said plants are not exclusively obtained by means of an essentially biological process.

In some embodiments, provided herein are methods of selecting a cucumber plant exhibiting resistance to *Cucumber mosaic virus* (CMV). In certain embodiments, said methods comprise screening one or more plants with at least one nucleic acid marker to detect a polymorphism genetically linked to CMV resistance, and selecting one or more plants comprising said polymorphism genetically linked to CMV resistance. In further embodiments, said selecting comprises detecting a marker locus within or genetically linked to a chromosomal segment flanked in the genome of said plant by: marker locus M1 (SEQ ID NO: 11) and marker locus M2 (SEQ ID NO:20) on chromosome 2; or marker locus M3 (SEQ ID NO:1) and marker locus M4 (SEQ ID NO: 10) on chromosome 6. In other embodiments, said methods comprise: a) screening one or more plants with at least one nucleic acid marker to detect a polymorphism genetically linked to CMV resistance; and b) selecting one or more plants comprising a haplotype associated with CMV resistance, wherein the haplotype comprises a CMV resistance allele flanked in the genome of the plant by: marker locus M1 (SEQ ID NO:11) and marker locus M2 (SEQ ID NO:20) on chromosome 2; or marker locus M3 (SEQ ID NO:1) and marker locus M4 (SEQ ID NO: 10) on chromosome 6. In particular embodiments, selecting a cucumber plant exhibiting resistance to *Cucumber mosaic virus* (CMV) comprises molecular genetic techniques. For example, those of ordinary skill in the art viewing the present disclosure may use technical methods to select a cucumber plant exhibiting resistance to *Cucumber mosaic virus* (CMV) by screening one or more plants with at least one nucleic acid marker to detect a polymorphism genetically linked to CMV resistance.

In some embodiments, the present disclosure provides the markers shown in Table 1, which have been shown to be genetically linked to *Cucumber Mosaic Virus* resistance in plants.

In particular embodiments, plants comprising the CMV resistance alleles are provided. The CMV resistance alleles described herein provide robust resistance to *Cucumber Mosaic Virus.* Methods of producing the plants described herein are further provided. The disclosure further provides trait-linked markers which can be used to produce and/or select plants comprising the CMV resistance alleles on chromosomes 2, 5, and 6 conferring *Cucumber Mosaic Virus* resistance as described herein.

The present disclosure provides significant advancements in obtaining CMV resistance in cucumbers by identifying QTLs on chromosome 2 and chromosome 6. These QTLs are distinct from those known in the art. In addition, markers associated with the resistance alleles are provided, allowing the alleles to be accurately introgressed and tracked during plant breeding. As such, the present disclosure permits introgression of the disease resistance alleles into any desired cucumber genotype.

### I. Genomic Regions, Alleles, and Polymorphisms Associated With Cucumber mosaic virus Resistance in Cucumber Plants

Provided herein are introgressions of one or more alleles associated with *Cucumber mosaic virus* resistance, together with polymorphic nucleic acids and linked markers for tracking the introgressions during plant breeding.

Cucumber lines exhibiting *Cucumber mosaic virus* resistance are known in the art and may be used together with the trait-linked markers provided herein in accordance with the present disclosure. For example, cucumber line APDM3130438MO comprising the chromosomal segment on chromosomes 2, 5, and 6, wherein a representative sample of seed comprising the chromosomal segments has been deposited under ATCC Accession No. PTA-122638, can be used as a source for CMV resistance according to the disclosure. Similarly, cucumber line APDM3130430MO comprising the chromosomal segment on chromosomes 2, 5, and 6, wherein a representative sample of seed comprising the chromosomal segments has been deposited under ATCC Accession No. PTA-122639, can also be used as a source for CMV resistance according to the disclosure.

Using the improved genetic markers and the assays described herein, those skilled in the art are able to successfully produce and/or select plants comprising the *Cucumber mosaic virus* resistance alleles described herein, which confer increased resistance to CMV as compared to a plant not comprising the allele(s). In certain embodiments, provided herein are methods of introgressing into a plant a *Cucumber mosaic virus* resistance allele within a chromosomal segment flanked in the genome of the plant by marker locus M1 (SEQ ID NO: 11) and marker locus M2 (SEQ ID NO:20) on chromosome 2 and/or marker locus M3 (SEQ ID NO:1) and marker locus M4 (SEQ ID NO: 10) on chromosome 6. The present disclosure therefore represents a significant advance in the art.

### II. Introgression of Genomic Regions Associated with Cucumber mosaic virus Resistance

Marker-assisted introgression involves the transfer of a chromosomal region defined by one or more markers from a first genetic background to a second. Offspring of a cross that contain the introgressed genomic region can be identified by the combination of markers characteristic of the desired introgressed genomic region from a first genetic background and both linked and unlinked markers characteristic of the second genetic background.

Provided herein are accurate markers for identifying and tracking introgression of one or more of the genomic regions disclosed herein from a CMV resistant plant into a cultivated line. Further provided are markers for identifying and tracking the introgressions disclosed herein during plant breeding, including the markers set forth in Table 1.

Markers within or linked to any of the genomic intervals described herein may be useful in a variety of breeding efforts that include introgression of genomic regions associated with disease resistance into a desired genetic background. For example, a marker within 40 cM, 20 cM, 15 cM, 10 cM, 5cM, 2 cM, or 1 cM of a marker associated with disease resistance described herein can be used for marker-assisted introgression of genomic regions associated with a disease resistant phenotype.

Methods of producing, selecting, and identifying cucumber plants comprising one or more introgressed regions associated with a desired phenotype wherein at least 10%, 25%, 50%, 75%, 90%, or 99% of the remaining genomic sequences carry markers characteristic of the recurrent parent germplasm are also provided. Methods of producing, selecting, and identifying cucumber plants comprising an introgressed region comprising regions closely linked to or adjacent to the genomic regions and markers provided herein and associated with a disease resistance phenotype are also provided

### III. Development of Disease Resistant Cucumber Varieties

For most breeding objectives, commercial breeders work within germplasm that is "cultivated," "cultivated type," or "elite." These cultivated lines may be used as recurrent parents or as a source of recurrent parent alleles during breeding. Cultivated or elite germplasm is easier to breed because it generally performs well when evaluated for horticultural performance. Many cultivated cucumber types have been developed and are known in the art as being agronomically elite and appropriate for commercial cultivation. However, the performance advantage a cultivated germplasm provides can be offset by a lack of allelic diversity. Breeders generally accept this tradeoff because progress is faster when working with cultivated material than when breeding with genetically diverse sources.

In contrast, when cultivated germplasm is crossed with non-cultivated germplasm, a breeder can gain access to novel alleles from the non-cultivated type. Non-cultivated germplasm may be used as a source of donor alleles during breeding. However, this approach generally presents significant difficulties due to fertility problems associated with crosses between diverse lines, and negative linkage drag from the non-cultivated parent. For example, non-cultivated cucumber types can provide alleles associated with disease resistance. However, these non-cultivated types may have poor horticultural qualities such as poor fruit shape, agronomically unacceptable plant architecture, and/or necrosis.

The process of introgressing desirable resistance genes from non-cultivated lines into elite cultivated lines while avoiding problems with linkage drag or low heritability is a long and often arduous process. In deploying alleles derived from wild relatives it is often desirable to introduce a minimal or truncated introgression that provides the desired trait but lacks detrimental effects. To aid introgression reliable marker assays are preferable to phenotypic screens. Success is furthered by simplifying genetics for key attributes to allow focus on genetic gain for quantitative traits such as disease resistance. Moreover, the process of introgressing genomic regions from non-cultivated lines can be greatly facilitated by the availability of accurate markers for MAS.

One of skill in the art would therefore understand that the alleles, polymorphisms, and markers provided by the present disclosure allow the tracking and introduction of any of the genomic regions identified herein into any genetic background. In addition, the genomic regions associated with disease resistance disclosed herein can be introgressed from one genotype to another and tracked using MAS. Thus, the disclosure of accurate markers associated with disease resistance will facilitate the development of cucumber plants having beneficial phenotypes. For example, seed can be genotyped using the markers of the present disclosure to select for plants comprising desired genomic regions associated with disease resistance. Moreover, MAS allows identification of plants homozygous or heterozygous for a desired introgression.

Inter-species crosses can also result in suppressed recombination and plants with low fertility or fecundity. For example, suppressed recombination has been observed for the tomato nematode resistance geneMi, the *Mla* and *Mlg* genes in barley, the *Yr17* and Lr20 genes in wheat, the *Run1* gene in grapevine, and the *Rma* gene in peanut. Meiotic recombination is essential for classical breeding because it enables the transfer of favorable alleles across genetic backgrounds, the removal of deleterious genomic fragments, and pyramiding traits that are genetically tightly linked. Therefore suppressed recombination forces breeders to enlarge segregating populations for progeny screens in order to arrive at the desired genetic combination.

Phenotypic evaluation of large populations is time-consuming, resource-intensive and not reproducible in every environment. Marker-assisted selection offers a feasible alternative. Molecular assays designed to detect unique polymorphisms, such as SNPs, are versatile. However, they may fail to discriminate alleles within and among cucumber species in a single assay. Structural rearrangements of chromosomes such as deletions impair hybridization and extension of synthetically labeled oligonucleotides. In the case of duplication events, multiple copies are amplified in a single reaction without distinction. The development and validation of accurate and highly predictive markers are therefore essential for successful MAS breeding programs.

### IV. Marker Assisted Breeding and Genetic Engineering Techniques

Genetic markers that can be used in the practice of the present invention include, but are not limited to, restriction fragment length polymorphisms (RFLPs), amplified fragment length polymorphisms (AFLPs), simple sequence repeats (SSRs), simple sequence length polymorphisms (SSLPs), single nucleotide polymorphisms (SNPs), insertion/deletion polymorphisms (Indels), variable number tandem repeats (VNTRs), and random amplified polymorphic DNA (RAPD), isozymes, and other markers known to those skilled in the art. Marker discovery and development in crop plants provides the initial framework for applications to marker-assisted breeding activities (U.S. Patent Pub. Nos.: 2005/0204780, 2005/0216545, 2005/0218305, and 2006/00504538). The resulting "genetic map" is the representation of the relative position of characterized loci (polymorphic nucleic acid markers or any other locus for which alleles can be identified) to each other.

Polymorphisms comprising as little as a single nucleotide change can be assayed in a number of ways. For example, detection can be made by electrophoretic techniques including a single strand conformational polymorphism (Orita, et al. (1989) Genomics, 8(2), 271-278), denaturing gradient gel electrophoresis (Myers (1985) EPO 0273085), or cleavage fragment length polymorphisms (Life Technologies, Inc., Gaithersburg, MD), but the widespread availability of DNA sequencing often makes it easier to simply sequence amplified products directly. Once the polymorphic sequence difference is known, rapid assays can be designed for progeny testing, typically involving some version of PCR amplification of specific alleles (PASA; Sommer, et al. (1992) Biotechniques 12(1), 82-87), or PCR amplification of multiple specific alleles (PAMSA; Dutton and Sommer (1991) Biotechniques, 11(6), 700-7002).

Polymorphic markers serve as useful tools for assaying plants for determining the degree of identity of lines or varieties (U.S. Patent No. 6,207,367). These markers form the basis for determining associations with phenotypes and can be used to drive genetic gain. In certain embodiments, polymorphic nucleic acids can be used to detect in a cucumber plant a genotype associated with disease resistance, identify a cucumber plant with a genotype associated with disease resistance, and to select a cucumber plant with a genotype associated with disease resistance. In certain embodiments of methods described, polymorphic nucleic acids can be used to produce a cucumber plant that comprises in its genome an introgressed locus associated with disease resistance. In certain embodiments, polymorphic nucleic acids can be used to breed progeny cucumber plants comprising a locus or loci associated with disease resistance.

Genetic markers may include "dominant" or "codominant" markers. "Codominant" markers reveal the presence of two or more alleles (two per diploid individual). "Dominant" markers reveal the presence of only a single allele. Markers are preferably inherited in codominant fashion so that the presence of both alleles at a diploid locus, or multiple alleles in triploid or tetraploid loci, are readily detectable, and they are free of environmental variation, *i.e*., their heritability is 1. A marker genotype typically comprises two marker alleles at each locus in a diploid organism. The marker allelic composition of each locus can be either homozygous or heterozygous. Homozygosity is a condition where both alleles at a locus are characterized by the same nucleotide sequence. Heterozygosity refers to a condition where the two alleles at a locus are different.

Nucleic acid-based analyses for determining the presence or absence of the genetic polymorphism (*i.e.* for genotyping) can be used in breeding programs for identification, selection, introgression, and the like. A wide variety of genetic markers for the analysis of genetic polymorphisms are available and known to those of skill in the art. The analysis may be used to select for genes, portions of genes, QTL, alleles, or genomic regions that comprise or are linked to a genetic marker that is linked to or associated with disease resistance in cucumber plants.

As used herein, nucleic acid analysis methods include, but are not limited to, PCR-based detection methods (for example, TaqMan assays), microarray methods, mass spectrometry-based methods and/or nucleic acid sequencing methods, including whole genome sequencing. In certain embodiments, the detection of polymorphic sites in a sample of DNA, RNA, or cDNA may be facilitated through the use of nucleic acid amplification methods. Such methods specifically increase the concentration of polynucleotides that span the polymorphic site, or include that site and sequences located either distal or proximal to it. Such amplified molecules can be readily detected by gel electrophoresis, fluorescence detection methods, or other means.

One method of achieving such amplification employs the polymerase chain reaction (PCR) (Mullis et al. (1986) Cold Spring Harbor Symp. Quant. Biol. 51:263-273; European Patent 50,424; European Patent 84,796; European Patent 258,017; European Patent 237,362; European Patent 201,184; U.S. Patent 4,683,202; U.S. Patent 4,582,788; and U.S. Patent 4,683,194), using primer pairs that are capable of hybridizing to the proximal sequences that define a polymorphism in its double-stranded form. Methods for typing DNA based on mass spectrometry can also be used. Such methods are disclosed in US Patents 6,613,509 and 6,503,710, and references found therein.

Polymorphisms in DNA sequences can be detected or typed by a variety of effective methods well known in the art including, but not limited to, those disclosed in U.S. Patent Nos. 5,468,613, 5,217,863; 5,210,015; 5,876,930; 6,030,787; 6,004,744; 6,013,431; 5,595,890; 5,762,876; 5,945,283; 5,468,613; 6,090,558; 5,800,944; 5,616,464; 7,312,039; 7,238,476; 7,297,485; 7,282,355; 7,270,981 and 7,250,252 all of which are incorporated herein by reference in their entirety. However, the compositions and methods of the present disclosure can be used in conjunction with any polymorphism typing method to detect polymorphisms in genomic DNA samples. These genomic DNA samples used include but are not limited to, genomic DNA isolated directly from a plant, cloned genomic DNA, or amplified genomic DNA.

For instance, polymorphisms in DNA sequences can be detected by hybridization to allele-specific oligonucleotide (ASO) probes as disclosed in U.S. Patent Nos. 5,468,613 and 5,217,863. U.S. Patent No. 5,468,613 discloses allele specific oligonucleotide hybridizations where single or multiple nucleotide variations in nucleic acid sequence can be detected in nucleic acids by a process in which the sequence containing the nucleotide variation is amplified, spotted on a membrane and treated with a labeled sequence-specific oligonucleotide probe.

Target nucleic acid sequence can also be detected by probe ligation methods, for example as disclosed in U.S. Patent No. 5,800,944 where sequence of interest is amplified and hybridized to probes followed by ligation to detect a labeled part of the probe.

Microarrays can also be used for polymorphism detection, wherein oligonucleotide probe sets are assembled in an overlapping fashion to represent a single sequence such that a difference in the target sequence at one point would result in partial probe hybridization (Borevitz et al., Genome Res. 13:513-523 (2003); Cui et al., Bioinformatics 21:3852-3858 (2005). On any one microarray, it is expected there will be a plurality of target sequences, which may represent genes and/or noncoding regions wherein each target sequence is represented by a series of overlapping oligonucleotides, rather than by a single probe. This platform provides for high throughput screening of a plurality of polymorphisms. Typing of target sequences by microarray-based methods is described in US Patents 6,799,122; 6,913,879; and 6,996,476.

Other methods for detecting SNPs and Indels include single base extension (SBE) methods. Examples of SBE methods include, but are not limited, to those disclosed in U.S. Patent Nos. 6,004,744; 6,013,431; 5,595,890; 5,762,876; and 5,945,283.

In another method for detecting polymorphisms, SNPs and Indels can be detected by methods disclosed in U.S. Patent Nos. 5,210,015; 5,876,930; and 6,030,787 in which an oligonucleotide probe having a 5' fluorescent reporter dye and a 3' quencher dye covalently linked to the 5' and 3' ends of the probe. When the probe is intact, the proximity of the reporter dye to the quencher dye results in the suppression of the reporter dye fluorescence, *e.g.* by Forster-type energy transfer. During PCR, forward and reverse primers hybridize to a specific sequence of the target DNA flanking a polymorphism while the hybridization probe hybridizes to polymorphism-containing sequence within the amplified PCR product. In the subsequent PCR cycle DNA polymerase with 5' → 3' exonuclease activity cleaves the probe and separates the reporter dye from the quencher dye resulting in increased fluorescence of the reporter.

In another embodiment, a locus or loci of interest can be directly sequenced using nucleic acid sequencing technologies. Methods for nucleic acid sequencing are known in the art and include technologies provided by 454 Life Sciences (Branford, CT), Agencourt Bioscience (Beverly, MA), Applied Biosystems (Foster City, CA), LI-COR Biosciences (Lincoln, NE), NimbleGen Systems (Madison, WI), Illumina (San Diego, CA), and VisiGen Biotechnologies (Houston, TX). Such nucleic acid sequencing technologies comprise formats such as parallel bead arrays, sequencing by ligation, capillary electrophoresis, electronic microchips, "biochips," microarrays, parallel microchips, and single-molecule arrays.

Various genetic engineering technologies have been developed and may be used by those of skill in the art to introduce traits in plants. In certain aspects, traits are introduced into cucumber plants *via* altering or introducing a single genetic locus or transgene into the genome of a variety or progenitor thereof. Methods of genetic engineering to modify, delete, or insert genes and polynucleotides into the genomic DNA of plants are well-known in the art.

In specific embodiments, improved cucumber lines can be created through the site-specific modification of a plant genome. Methods of genetic engineering include, for example, utilizing sequence-specific nucleases such as zinc-finger nucleases (*see*, for example, U.S. Pat. Appl. Pub. No. 2011-0203012); engineered or native meganucleases; TALE-endonucleases (*see*, for example, U.S. Pat. Nos. 8,586,363 and 9,181,535); and RNA-guided endonucleases, such as those of the CRISPR/Cas systems (*see*, for example, U.S. Pat. Nos. 8,697,359 and 8,771,945 and U.S. Pat. Appl. Pub. No. 2014-0068797). One embodiment of the disclosure thus relates to utilizing a nuclease or any associated protein to carry out genome modification. This nuclease could be provided heterologously within donor template DNA for templated-genomic editing or in a separate molecule or vector. A recombinant DNA construct may also comprise a sequence encoding one or more guide RNAs to direct the nuclease to the site within the plant genome to be modified. Further methods for altering or introducing a single genetic locus include, for example, utilizing single-stranded oligonucleotides to introduce base pair modifications in a cucumber plant genome (*see*, for example Sauer et al., Plant Physiol, 170(4):1917-1928, 2016).

Methods for site-directed alteration or introduction of a single genetic locus are well-known in the art and include those that utilize sequence-specific nucleases, such as the aforementioned, or complexes of proteins and guide-RNA that cut genomic DNA to produce a double-strand break (DSB) or nick at a genetic locus. As is well-understood in the art, during the process of repairing the DSB or nick introduced by the nuclease enzyme, a donor template, transgene, or expression cassette polynucleotide may become integrated into the genome at the site of the DSB or nick. The presence of homology arms in the DNA to be integrated may promote the adoption and targeting of the insertion sequence into the plant genome during the repair process through homologous recombination or non-homologous end joining (NHEJ).

In another embodiment of the present disclosure, genetic transformation may be used to insert a selected transgene into a plant or may, alternatively, be used for the preparation of transgenes which can be introduced by backcrossing. Methods for the transformation of plants that are well-known to those of skill in the art and applicable to many crop species include, but are not limited to, electroporation, microprojectile bombardment, *Agrobacterium-mediated* transformation, and direct DNA uptake by protoplasts.

To effect transformation by electroporation, one may employ either friable tissues, such as a suspension culture of cells or embryogenic callus or alternatively one may transform immature embryos or other organized tissue directly. In this technique, one would partially degrade the cell walls of the chosen cells by exposing them to pectin-degrading enzymes (pectolyases) or mechanically wound tissues in a controlled manner.

An efficient method for delivering transforming DNA segments to plant cells is microprojectile bombardment. In this method, particles are coated with nucleic acids and delivered into cells by a propelling force. Exemplary particles include those comprised of tungsten, platinum, and preferably, gold. For the bombardment, cells in suspension are concentrated on filters or solid culture medium. Alternatively, immature embryos or other target cells may be arranged on solid culture medium. The cells to be bombarded are positioned at an appropriate distance below the macroprojectile stopping plate.

An illustrative embodiment of a method for delivering DNA into plant cells by acceleration is the Biolistics Particle Delivery System, which can be used to propel particles coated with DNA or cells through a screen, such as a stainless steel or Nytex screen, onto a surface covered with target cells. The screen disperses the particles so that they are not delivered to the recipient cells in large aggregates. Microprojectile bombardment techniques are widely applicable, and may be used to transform virtually any plant species.

*Agrobacterium-*mediated transfer is another widely applicable system for introducing gene loci into plant cells. An advantage of the technique is that DNA can be introduced into whole plant tissues, thereby bypassing the need for regeneration of an intact plant from a protoplast. Modern *Agrobacterium* transformation vectors are capable of replication in *E. coli* as well as *Agrobacterium,* allowing for convenient manipulations (Klee et al., Nat. Biotechnol., 3(7):637-642, 1985). Moreover, recent technological advances in vectors for *Agrobacterium-*mediated gene transfer have improved the arrangement of genes and restriction sites in the vectors to facilitate the construction of vectors capable of expressing various polypeptide coding genes. The vectors described have convenient multi-linker regions flanked by a promoter and a polyadenylation site for direct expression of inserted polypeptide coding genes. Additionally, *Agrobacterium* containing both armed and disarmed Ti genes can be used for transformation.

In those plant strains where *Agrobacterium*-mediated transformation is efficient, it is the method of choice because of the facile and defined nature of the gene locus transfer. The use of *Agrobacterium-*mediated plant integrating vectors to introduce DNA into plant cells is well known in the art (Fraley et al., Nat. Biotechnol., 3:629-635, 1985; U.S. Patent No. 5,563,055).

Transformation of plant protoplasts also can be achieved using methods based on calcium phosphate precipitation, polyethylene glycol treatment, electroporation, and combinations of these treatments *(see,* for example, Potrykus et al., Mol. Gen. Genet., 199: 183-188, 1985; Omirulleh et al., Plant Mol. Biol., 21(3):415-428, 1993; Fromm et al., Nature, 312:791-793, 1986; Uchimiya et al., Mol. Gen. Genet., 204:204, 1986; Marcotte et al., Nature, 335:454, 1988). Transformation of plants and expression of foreign genetic elements is exemplified in Choi et al. (Plant Cell Rep., 13:344-348, 1994), and Ellul et al. (Theor. Appl. Genet., 107:462-469, 2003).

### V. Definitions

The following definitions are provided to better define the present invention and to guide those of ordinary skill in the art in the practice of the present invention. Unless otherwise noted, terms are to be understood according to conventional usage by those of ordinary skill in the relevant art.

As used herein, the term "plant" includes plant cells, plant protoplasts, plant cells of tissue culture from which cucumber plants can be regenerated, plant calli, plant clumps and plant cells that are intact in plants or parts of plants such as pollen, flowers, seeds, leaves, stems, and the like.

As used herein, the term "population" means a genetically heterogeneous collection of plants that share a common parental derivation.

As used herein, the terms "variety" and "cultivar" mean a group of similar plants that by their genetic pedigrees and performance can be identified from other varieties within the same species.

As used herein, an "allele" refers to one of two or more alternative forms of a genomic sequence at a given locus on a chromosome.

A "quantitative trait locus" (QTL) is a chromosomal location that encodes for at least a first allele that affects the expressivity of a phenotype.

As used herein, a "marker" means a detectable characteristic that can be used to discriminate between organisms. Examples of such characteristics include, but are not limited to, genetic markers, biochemical markers, metabolites, morphological characteristics, and agronomic characteristics.

As used herein, the term "phenotype" means the detectable characteristics of a cell or organism that can be influenced by gene expression.

As used herein, the term "genotype" means the specific allelic makeup of a plant.

As used herein, "elite" or "cultivated" variety means any variety that has resulted from breeding and selection for superior agronomic performance. An "elite plant" refers to a plant belonging to an elite variety. Numerous elite varieties are available and known to those of skill in the art of cucumber breeding. An "elite population" is an assortment of elite individuals or varieties that can be used to represent the state of the art in terms of agronomically superior genotypes of a given crop species, such as cucumber. Similarly, an "elite germplasm" or elite strain of germplasm is an agronomically superior germplasm.

As used herein, the term "introgressed," when used in reference to a genetic locus, refers to a genetic locus that has been introduced into a new genetic background, such as through backcrossing. Introgression of a genetic locus can be achieved through plant breeding methods and/or by molecular genetic methods. Such molecular genetic methods include, but are not limited to, various plant transformation techniques and/or methods that provide for homologous recombination, non-homologous recombination, site-specific recombination, and/or genomic modifications that provide for locus substitution or locus conversion.

As used herein, the terms "recombinant" or "recombined" in the context of a chromosomal segment refer to recombinant DNA sequences comprising one or more genetic loci in a configuration in which they are not found in nature, for example as a result of a recombination event between homologous chromosomes during meiosis.

As used herein, the term "linked," when used in the context of nucleic acid markers and/or genomic regions, means that the markers and/or genomic regions are located on the same linkage group or chromosome such that they tend to segregate together at meiosis.

As used herein, "tolerance locus" means a locus associated with tolerance or resistance to disease. For instance, a tolerance locus according to the present disclosure may, in one embodiment, control tolerance or susceptibility to CMV.

As used herein, "tolerance" or "improved tolerance" in a plant refers to the ability of the plant to perform well, for example by maintaining yield, under disease conditions. Tolerance may also refer to the ability of a plant to maintain a plant vigor phenotype under disease conditions. Tolerance is a relative term, indicating that a "tolerant" plant is more able to maintain performance compared to a different (less tolerant) plant (e.g. a different plant variety) grown in similar disease conditions. One of skill will appreciate that plant tolerance to disease conditions varies widely, and can represent a spectrum of more-tolerant or less-tolerant phenotypes. However, by simple observation, one of skill can generally determine the relative tolerance of different plants, plant varieties, or plant families under disease conditions, and furthermore, will also recognize the phenotypic gradations of "tolerance."

As used herein "resistance" or "improved resistance" in a plant to disease conditions is an indication that the plant is more able to reduce disease burden than a non-resistant or less resistant plant. Resistance is a relative term, indicating that a "resistant" plant is more able to reduce disease burden compared to a different (less resistant) plant (e.g., a different plant variety) grown in similar disease conditions. One of skill will appreciate that plant resistance to disease conditions varies widely, and can represent a spectrum of more-resistant or less-resistant phenotypes. However, by simple observation, one of skill can generally determine the relative resistance of different plants, plant varieties, or plant families under disease conditions, and furthermore, will also recognize the phenotypic gradations of "resistant."

As used herein, "resistance allele" means the nucleic acid sequence associated with tolerance or resistance to disease. The use of the term "a resistance allele" does not exclude a genomic region that comprises more than one gene or other genetic factor. Specifically, a "disease resistance allele" can denote a haplotype allele within a haplotype window or genomic region wherein a phenotype associated with the haplotype allele can be disease resistance. A haplotype window is a contiguous genomic region that can be defined, and tracked, with a set of one or more polymorphic markers wherein the polymorphisms indicate identity by descent. A haplotype within that window can be defined by the unique fingerprint of alleles at each marker. When all the alleles present at a given locus on a chromosome are the same, that plant is homozygous at that locus. If the alleles present at a given locus on a chromosome differ, that plant is heterozygous at that locus. Plants may be homozygous or heterozygous at any particular resistance locus or for a particular polymorphic marker.

As used herein, "polymorphism" means the presence of one or more variations of a nucleic acid sequence at one or more loci in a population of one or more individuals. The variation may comprise but is not limited to one or more base changes, the insertion of one or more nucleotides or the deletion of one or more nucleotides. A polymorphism may arise from random processes in nucleic acid replication, through mutagenesis, as a result of mobile genomic elements, from copy number variation and during the process of meiosis, such as unequal crossing over, genome duplication and chromosome breaks and fusions. The variation can be commonly found or may exist at low frequency within a population, the former having greater utility in general plant breeding and the latter may be associated with rare but important phenotypic variation. Useful polymorphisms may include single nucleotide polymorphisms (SNPs), insertions or deletions in DNA sequence (Indels), simple sequence repeats of DNA sequence (SSRs) a restriction fragment length polymorphism, and a tag SNP. A genetic marker, a gene, a DNA-derived sequence, a haplotype, a RNA-derived sequence, a promoter, a 5' untranslated region of a gene, a 3' untranslated region of a gene, microRNA, siRNA, a QTL, a satellite marker, a transgene, mRNA, ds mRNA, a transcriptional profile, and a methylation pattern may comprise polymorphisms

As used herein, the term "haplotype" means a chromosomal region within a haplotype window defined by at least one polymorphic molecular marker. The unique marker fingerprint combinations in each haplotype window define individual haplotypes for that window. Further, changes in a haplotype, brought about by recombination for example, may result in the modification of a haplotype so that it comprises only a portion of the original (parental) haplotype operably linked to the trait, for example, via physical linkage to a gene, QTL, or transgene. Any such change in a haplotype would be included in the definition of what constitutes a haplotype so long as the functional integrity of that genomic region is unchanged or improved.

As used herein, the term "haplotype window" means a chromosomal region that is established by statistical analyses known to those of skill in the art and is in linkage disequilibrium. Thus, identity by state between two inbred individuals (or two gametes) at one or more molecular marker loci located within this region is taken as evidence of identity-by-descent of the entire region. Each haplotype window includes at least one polymorphic molecular marker. Haplotype windows can be mapped along each chromosome in the genome. Haplotype windows are not fixed *per se* and, given the ever-increasing density of molecular markers, this disclosure anticipates the number and size of haplotype windows to evolve, with the number of windows increasing and their respective sizes decreasing, thus resulting in an ever-increasing degree confidence in ascertaining identity by descent based on the identity by state at the marker loci.

The term "about" is used to indicate that a value includes the standard deviation of error for the device or method being employed to determine the value. The use of the term "or" in the claims is used to mean "and/or" unless explicitly indicated to refer to alternatives only or the alternatives are mutually exclusive, although the disclosure supports a definition that refers to only alternatives and to "and/or." When used in conjunction with the word "comprising" or other open language in the claims, the words "a" and "an" denote "one or more," unless specifically noted. The terms "comprise," "have" and "include" are open-ended linking verbs. Any forms or tenses of one or more of these verbs, such as "comprises," "comprising," "has," "having," "includes" and "including," are also open-ended. For example, any method that "comprises," "has" or "includes" one or more steps is not limited to possessing only those one or more steps and also covers other unlisted steps. Similarly, any plant that "comprises," "has" or "includes" one or more traits is not limited to possessing only those one or more traits and covers other unlisted traits.

### VI. Deposit Information

A deposit was made of at least 2500 seeds of cucumber line APDM3130438MO, which comprises the chromosomal segments on chromosomes 2, 5, and 6 as described herein. The deposit was made with the American Type Culture Collection (ATCC), 10801 University Blvd., Manassas, VA 20110-2209. The deposit is assigned ATCC Accession No. PTA-122638, and the date of deposit was October 22, 2015. Access to the deposit will be available during the pendency of the application to persons entitled thereto upon request. The deposit has been accepted under the Budapest Treaty and will be maintained in the ATCC Depository, which is a public depository, for a period of 30 years, or 5 years after the most recent request, or for the enforceable life of the patent, whichever is longer, and will be replaced if nonviable during that period. Applicant does not waive any infringement of their rights granted under this patent or any other form of variety protection, including the Plant Variety Protection Act (7 U.S.C. 2321 *et seq*.).

A deposit was made of at least 2500 seeds of cucumber line APDM3130430MO, which comprises the chromosomal segments on chromosomes 2, 5, and 6 as described herein. The deposit was made with the American Type Culture Collection (ATCC), 10801 University Blvd., Manassas, VA 20110-2209. The deposit is assigned ATCC Accession No. PTA-122639, and the date of deposit was October 22, 2015. Access to the deposit will be available during the pendency of the application to persons entitled thereto upon request. The deposit has been accepted under the Budapest Treaty and will be maintained in the ATCC Depository, which is a public depository, for a period of 30 years, or 5 years after the most recent request, or for the enforceable life of the patent, whichever is longer, and will be replaced if nonviable during that period. Applicant does not waive any infringement of their rights granted under this patent or any other form of variety protection, including the Plant Variety Protection Act (7 U.S.C. 2321 *et seq*.).

### Examples

### Example 1. Selection, Inoculation, and Scoring of Cucumber Panel for Genome Wide Association Studies

As described herein, Genome Wide Association Studies (GWAS) were conducted in order to map QTLs by associating a genetic variant with a phenotypic variation. A large diversity panel comprising approximately 200 elite lines, 50 landraces, and 20 hybrids (including resistance controls) was evaluated as described herein (summarized in FIG. 2).

To produce the CMV inoculums used in the study, CMV isolates were separately and mechanically inoculated in plants of the 'Corona' cucumber variety ten days before the diversity panel inoculation. Two isolates (1: 1 concentration) were co-inoculated, with one strain developing symptoms in cold conditions and the other one in warm conditions to ensure symptom observation. When cotyledons were fully expanded, around one-week post-sowing, 10g of Corona leaves were mixed in 50 ml of phosphate buffer Na₂HPO₄-0.03M + Sodium diethyldithiocarbamate 0.2%, and 2.5g of charcoal and carborundum. Inoculums were kept in ice during inoculation (4°C) and cotyledons were hand-rubbed twice, by two different operators to avoid inoculation escape. Inoculum was refreshed between each genotype.

The diversity panel was inoculated with CMV in a single greenhouse compartment in Bergschenhoek, the Netherlands. The phenotypic response to the virus was evaluated with three randomized complete blocks of five plants per accession. The plant response was scored in nine ordered classes. The symptom severity (SS) was individually scored around 14 days post-inoculation (DPI) when intermediate and susceptible checks displayed the expected symptoms. We used the following notation scale: 1 - no symptoms, 3 - one to three symptom spots on one leave, 5 - limited symptoms areas on young leaves, 7 - more numerous symptoms on young and old leaves, 9 - plant growth halted, development of obvious severe symptoms (leaf mosaic, vein yellowing, deformation, bubbling depending on the virus). Even scores were attributed when plants displayed intermediate symptoms between uneven scores. All scores higher or lower than 2 standard deviations within each accession were considered as outliers and discarded from the phenotypic dataset.

An aggregated score for each accession was obtained using a generalized linear model to deal with the phenotype discontinuity and called Poisson BLUP (PoP). Hybrid controls gave the expected phenotypes.

### Example 2. Genome Wide Association Studies of Cucumber Panel

Leaf samples from three plants were bulked for each elite line and landrace and genotyped by ILLUMINA 150 pair-end. The CCL public genome, assembled via SMRT from long-read sequences (Li et al. 2019), was used for sequence alignment and variant calling. Variant discovery was performed using GATK4 Best Practices (Van der Auwera et O'Connor 2020), first by calling individual sample SNPs, followed by joint genotyping on the population. All biallelic and multiallelic SNPs, as well as InDels, with a depth equal or superior to 20X and a phred score superior to 20 for each line were collected, resulting in a genetic dataset of 2,081,122 biallelic SNPs, 28,162 multiallelic SNPs and 30,557 InDels. The SNPs were then filtered for QD (Variant Confidence/Quality by Dept) >18. SNPs exhibiting a missing rate above 10%, a heterozygous rate above 15% and a minor allele frequency (MAF) under 3.125% (~8 homozygous genotypes), were discarded. InDels and multi-allelic SNPs were also discarded. Four lines, including three landraces, exhibiting more than 30% missing data and 30% heterozygous rate were discarded from the diversity panel.

The genotyping protocol, SNP calling, and filtering described resulted in a SNP matrix called M_{NA10} which contained 250 lines and 1,271,848 SNPs. We estimated the LD extent by randomly selecting 10K SNP on each chromosome. We calculated the r² between all SNPs in a 1 Mb window and performed a loess regression with a span of 0.2. The LD extent was assessed when a r² =0.2 was reached. The population genetic structure matrix was designed via the sparse nonnegative matrix factorization (sNMF) algorithm as described in Frichot *et al.* 2014.

From M_{NA10}, a genotype matrix was derived by removing SNPs with at least one NA and with a MAF< 3.1%, resulting in a matrix called M_{NA00} containing a total of 376,151 SNPs. The MLMM model previously described (Segura et al. 2012) and implemented in the R package mlmm was used to perform GWAS on the 376,151 SNPs. MLMM is a multistep approach, each step being a GWAS using the MLM described below but complemented with each top SNP of previous steps, included in the model as fixed effects. Five models were tested, e.g., Kinship (K), genetic structure (Q5), genetic structure (Q9), Kinship + genetic structure (KQ5), Kinship + genetic structure (KQ9). The last MLMM step was defined when no further SNPs were found below the Bonferroni threshold or when no phenotypic variance explained by genetic remained. Bonferroni significance threshold was set up with a genome wide error risk α=0.05 corrected by the number of independent SNPs (Gao 2011). We detected 115,391 independent SNPs with Gao's method using a sliding window of 100kb. It set the Bonferroni threshold for α=0.05 at 4.3x10-7 (-log10 = 6,4) and for α=0.01 at 8.7x10-7 (-log10 = 7,1) for GWAS analyses. The different models implemented were compared for their capacity to balance false positive and negative rates via QQ plots generated from qqman R package (Turner 2014). The null model with only the structure effects was also tested to estimate the part of phenotypic variance explained by genetic groups.

The accessions within the panel evaluated belonged to at least five horticultural groups. Their genetic structure was investigated with the `snmf a algorithm (Frichot et al. 2014). Five groups matched with the horticultural groups (FIG. 3) while nine groups matched with breeding programs (FIG. 4). For the nine groups (FIG. 4), landraces were pooled into two genetic groups, one with accessions from Asia and the other one with accessions from Europe and America. The seven other groups dealt with elite lines from different horticultural groups: Long-Dutch (group 1), pickles (groups 4 and 5), slicers (groups 6 and 7) and Beit-alpha (groups 8 and 9). A hierarchical clustering of the kinship matrix confirmed the nine-group genetic structure. Accessions from the genetic group 2, *i.e*., Asian landraces showed strong kinship coefficients within their group (~3) compared to the rest of the population highlighting the presence of specific alleles in this group. This group also had low kinship coefficients (~-1) with some accessions from group 1 (Long-Dutch), highlighting their genetic distance with these elite lines.

The nine-group genetic structure explained 49% of the phenotypic variance (shown in grey in FIG. 5). The phenotypic variance was heterogenous among the nine genetic groups and the distribution of resistant accessions was dissected (FIG. 3C) since it can affect GWAS results. The landraces from group 2 were highly resistant to CMV, while the landraces from group 3 were highly susceptible to CMV. Remarkably, elite-line genetic groups displayed more individuals admixed with landraces from the group 2 than from the group 3 (FIG. 3C), suggesting that landraces from group 2 were used as resistance donors. Then, resistant alleles from group 2, might be frequent enough in the panel to reach the MAF threshold. Elite lines from genetic groups 4, 5, 6, and 9 were highly resistant to CMV while accessions from other groups displayed phenotypic variance.

In brief, an iterative GWAS (MLMM), that introduced the most significant SNPs from previous steps as fixed effect was used. Of the five models tested using PoPs as aggregated phenotypes (Q5, Q9, K, KQ5 and KQ9), the model including both the kinship and nine genetic groups (KQ9), collected in the MLMM first step resulted in a p-values curve that best fit the bisector on the qqplot. This model reflected a quasi-uniform distribution of the p-values for most SNPs, as expected under the hypothesis that only few markers are likely associated to QTLs.

Output obtained directly from the GWAS defined a top SNP on chromosome 5 and QTL intervals on chromosomes 2 and 6. In particular, the top SNP on chr5 was located at 7,197,002 bp; the interval located on chr2 spanned from 9,183,788 bp to 9,417,996 bp; and the interval located on chr6 spanned from 30,018,048 bp to 30,084,128 bp; each location made with referenced of the public cucumber genome map version Cucumber Chinese Long' (CCL) landrace. Subsequent post-GWAS analysis performed on M_{NA10} (1,271,848 SNPs), which takes into consideration the linkage disequilibrium of the peaks identified, detected three QTLs for CMV resistance (FIG. 6), all having a top SNP MAF>0.4. They explained from 7 to 11% of the PV (FIG. 5; in middle grey shade). Furthermore, 75% of the phenotypic variance could be explained by combining the SNPs on chromosomes 2, 5, and 6. The QTL located on chr2 was 1.9 Mb wide, the QTL on chr5 was 1.1 Mb, and the QTL located on one end of the chr6 was 1.6 Mb QTL wide. Additional markers that associated with the CMV resistance QTLs described (M1-M27) were also identified and are provided in Table 1 below.

**Table 1. List of markers and favorable alleles at each marker for tracking resistance QTLs.**

| **Marker name** | **Chr.** | **Public position or [interval] SNP (bp)** | **Marker size (bp)** | **SNP position in marker (bp)** | **SNP change** | **Favorable allele** | **Marker sequence (SEQ ID NO)** | **Fwd primer (SEQ ID NO)** | **Rev primer (SEQ ID NO)** |
|---|---|---|---|---|---|---|---|---|---|
| M1 | 2 | 7,671,123 | 2000 | 1000 | [T/C] | T | 11 | | |
| M2 | 2 | 9,602,331 | 2000 | 1000 | [G/A] | G | 20 | | |
| M3 | 6 | 29,444,900 | 2000 | 1000 | [A/T] | A | 1 | | |
| M4 | 6 | 31,058,514 | 2000 | 1000 | [C/A] | C | 10 | | |
| M5 | 2 | 7,885,709 | 200 | 100 | [G/A] | G | 12 | | |
| M6 | 2 | 8,099,630 | 200 | 99 | [T/C] | T | 13 | | |
| M7 | 2 | 8,314,916 | 200 | 96 | [T/C] | T | 14 | | |
| M8 | 2 | 8,517,088 | 200 | 101 | [G/A] | G | 15 | | |
| M9 | 2 | 8,745,783 | 200 | 101 | [G/A] | G | 16 | | |
| M10 | 2 | 8,958,596 | 200 | 101 | [G/A] | G | 17 | | |
| M11 | 2 | 9,173,190 | 200 | 98 | [T/C] | T | 18 | | |
| M12 | 2 | 9,387,861 | 200 | 101 | [C/A] | C | 19 | | |
| M13 | 6 | 29,625,182 | 200 | 100 | [T/C] | T | 2 | | |
| M14 | 6 | 29,851,680 | 200 | 99 | [T/C] | T | 3 | | |
| M15 | 6 | 29,978,956 | 200 | 99 | [C/G] | C | 4 | | |
| M16 | 6 | 30,165,530 | 200 | 99 | [A/G] | A | 5 | | |
| M17 | 6 | 30,347,998 | 200 | 101 | [C/G] | C | 6 | | |
| M18 | 6 | 30,520,715 | 200 | 100 | [A/C] | A | 7 | | |
| M19 | 6 | 30,622,852 | 200 | 100 | [T/G] | T | 8 | | |
| M20 | 6 | 30,746,021 | 200 | 100 | [G/A] | G | 9 | | |
| M21 | 5 | 6,284,008 | 2000 | 1000 | [C/T] | C | 29 | | |
| M22 | 5 | 7,437,769 | 2000 | 1002 | [T/C] | C | 30 | | |
| M23 | 5 | 7,193,467 | 200 | 100 | [A/T] | A | 31 | | |
| M24 | 2 | 9,183,788 | 200 | 98 | [T/C] | T | 32 | | |
| M25 | 2 | 9,417,996 | 200 | 101 | [G/C] | G | 33 | | |
| M26 | 6 | 30,018,048 | 200 | 101 | [C/T] | C | 34 | | |
| M27 | 6 | 30,084,128 | 200 | 101 | [A/G] | A | 35 | | |
| KT3164 24 | 5 | 7,213,313-7,213,420 | 107 | | | | | 21 | 22 |
| KT3164 25 | 5 | 7,219,839-7,220,382 | 543 | | | | | 23 | 24 |
| SSR9-56 | 6 | 7,678,605-7,678,775 | 170 | | | | | 25 | 26 |
| SSR11-177 | 6 | 9,707,081-9,707,252 | 171 | | | | | 27 | 28 |

### Example 3. Genome Wide Association Studies Map Two Clusters of Resistance and CMV Resistance Haplotypes

A hierarchical clustering of the local kinship was also implemented on the QTLs for CMV resistance in chr2, chr5, and chr6. It revealed several resistant haplotypes unequally distributed among genetic structure groups (FIGS. 7-9). Two resistant haplotypes were found in the QTL on chr2, one large segment from the reference genome CCL, a CMV resistant source, and one specific to the group 4 (FIG. 7). Several haplotypes in the QTL on chr6 were also observed (FIG. 8). Haplotype 2 was mainly present in resistant accessions from the groups 2, 4 and admixed accessions.

### Example 4. Introgression of Cucumber Mosaic Virus Resistance Loci

The QTLs on chromosomes 2 and 6 will be further validated using a large panel of cucumber lines and hybrids analogous to the diversity panel described in Example 1. The effect of each QTL as well as the combination of QTLs described herein will be validated using phenotypes for resistance to CMV and genotypes at the QTLs.

Additionally, the markers disclosed will be used to perform Marker Assisted Selection (MAS) for resistance to CMV. For example, cucumber breeders will use these molecular markers to create lines resistant to CMV by selecting the favorable allele(s) described herein. Stacking the CMV QTLs on chromosome 2, 6, and 5, will be performed to create lines outperforming lines carrying only one or two of the QTLs for CMV resistance.

The following represent further embodiments of the invention:
1. A method of selecting a cucumber plant exhibiting resistance to *Cucumber mosaic virus* (CMV), comprising:
   a) screening one or more plants with at least one nucleic acid marker to detect a polymorphism genetically linked to CMV resistance; and
   b) selecting one or more plants comprising a haplotype associated with CMV resistance, wherein the haplotype comprises a CMV resistance allele flanked in the genome of said plant by:
      marker locus M1 (SEQ ID NO:11) and marker locus M2 (SEQ ID NO:20) on chromosome 2; or
      marker locus M3 (SEQ ID NO:1) and marker locus M4 (SEQ ID NO: 10) on chromosome 6.
2. A method for identifying a cucumber plant comprising a *Cucumber mosaic virus* (CMV) resistance allele:
   (a) obtaining nucleic acids from at least a first cucumber plant; and
   (b) identifying in said nucleic acids the presence of at least a first genetic marker indicative of the presence of a chromosomal segment flanked in the genome of said plant by:
      marker locus M1 (SEQ ID NO:11) and marker locus M2 (SEQ ID NO:20) on chromosome 2; or
      marker locus M3 (SEQ ID NO: 1) and marker locus M4 (SEQ ID NO: 10) on chromosome 6
      wherein said CMV resistance allele confers to said plant increased resistance to CMV compared to a plant not comprising said allele.
3. A method for selecting a cucumber plant with increased resistance to *Cucumber mosaic virus* (CMV) comprising:
   selecting a plant from a population of plants, at least some of comprise a CMV resistance,
   wherein selecting comprises detecting a marker locus within or genetically linked to a chromosomal segment flanked in the genome of said plant by:
      marker locus M1 (SEQ ID NO:11) and marker locus M2 (SEQ ID NO:20) on chromosome 2; or
      marker locus M3 (SEQ ID NO:1) and marker locus M4 (SEQ ID NO:10) on chromosome 6.
4. A method of selecting a cucumber plant exhibiting resistance to *Cucumber mosaic virus* (CMV), comprising:
   a) obtaining a population of plants having a parent comprising resistance to CMV;
   b) screening said population with at least one nucleic acid marker to detect a polymorphism genetically linked to CMV resistance; and
   c) selecting from said population one or more plants comprising a haplotype associated with CMV resistance, wherein the haplotype comprises a CMV resistance allele flanked in the genome of said plant by:
      marker locus M1 (SEQ ID NO:11) and marker locus M2 (SEQ ID NO:20) on chromosome 2; or
      marker locus M3 (SEQ ID NO:1) and marker locus M4 (SEQ ID NO:10) on chromosome 6.
5. A method of producing a cucumber plant exhibiting resistance to *Cucumber mosaic virus* (CMV), comprising introgressing into a plant a *Cucumber mosaic virus* resistance allele within a chromosomal segment flanked in the genome of said plant by marker locus M1 (SEQ ID NO:11) and marker locus M2 (SEQ ID NO:20) on chromosome 2; or marker locus M3 (SEQ ID NO:1) and marker locus M4 (SEQ ID NO: 10) on chromosome 6; wherein said introgressed CMV resistance allele confers to said plant increased resistance to CMV compared to a plant not comprising said allele.
6. The method of any one of embodiments 1 to 5, wherein a representative sample of seed of a cucumber plant comprising resistance to CMV has been deposited under ATCC Accession No. PTA-122638.
7. The method of any one of embodiments 1 to 6, wherein the *Cucumber mosaic virus* resistance allele is further defined as:
   a) located within a chromosomal segment flanked in the genome of said plant by marker locus M24 (SEQ ID NO:32) and marker locus M25 (SEQ ID NO:33) on chromosome 2; or
   b) located within a chromosomal segment flanked in the genome of said plant by marker locus M26 (SEQ ID NO:34) and marker locus M27 (SEQ ID NO:35) on chromosome 6.
8. The method of any one of embodiments 1 to 7, wherein said chromosomal segment comprises a marker locus on chromosome 2 selected from the group consisting of marker locus M5 (SEQ ID NO: 12), marker locus M6 (SEQ ID NO: 13), marker locus M7 (SEQ ID NO:14), marker locus M8 (SEQ ID NO: 15), marker locus M9 (SEQ ID NO:16), marker locus M10 (SEQ ID NO:17), marker locus M11 (SEQ ID NO:18), and marker locus M12 (SEQ ID NO:19).
9. The method of any one of embodiments 1 to 8, wherein said chromosomal segment comprises a marker locus on chromosome 6 selected from the group consisting of marker locus M13 (SEQ ID NO:2), marker locus M14 (SEQ ID NO:3), marker locus M15 (SEQ ID NO:4), marker locus M16 (SEQ ID NO:5), marker locus M17 (SEQ ID NO:6), marker locus M18 (SEQ ID NO:7), marker locus M19 (SEQ ID NO:8), and marker locus M20 (SEQ ID NO: 9).
10. The method of embodiment 9, wherein selecting said progeny plant is further defined as detecting a marker locus within or genetically linked to a chromosomal segment flanked in the genome of said plant by:
   a) marker locus M24 (SEQ ID NO:32) and marker locus M25 (SEQ ID NO:33) on chromosome 2; or
   b) marker locus M26 (SEQ ID NO:34) and marker locus M27 (SEQ ID NO:35) on chromosome 6.
11. The method of any one of embodiments 1 to 10, wherein selecting a progeny plant comprises detecting nucleic acids comprising marker locus M5 (SEQ ID NO:12), marker locus M6 (SEQ ID NO:13), marker locus M7 (SEQ ID NO:14), marker locus M8 (SEQ ID NO:15), marker locus M9 (SEQ ID NO:16), marker locus M10 (SEQ ID NO:17), marker locus M11 (SEQ ID NO:18), marker locus M12 (SEQ ID NO:19), marker locus M13 (SEQ ID NO:2), marker locus M14 (SEQ ID NO:3), marker locus M15 (SEQ ID NO:4), marker locus M16 (SEQ ID NO:5), marker locus M17 (SEQ ID NO:6), marker locus M18 (SEQ ID NO:7), marker locus M19 (SEQ ID NO:8), or marker locus M20 (SEQ ID NO:9).
12. The method of any one of embodiments 1 to 11, wherein selecting said plant comprises:
   (a) detecting a marker locus within or genetically linked to a chromosomal segment flanked in the genome of said plant by marker locus M1 (SEQ ID NO:11) and marker locus M2 (SEQ ID NO:20) on chromosome 2;
   (b) detecting a marker locus within or genetically linked to a chromosomal segment flanked in the genome of said plant by marker locus M3 (SEQ ID NO:1) and marker locus M4 (SEQ ID NO:10) on chromosome 6; or
   (c) detecting at least one polymorphism at a locus selected from the group consisting of marker locus marker locus M5 (SEQ ID NO:12), marker locus M6 (SEQ ID NO:13), marker locus M7 (SEQ ID NO:14), marker locus M8 (SEQ ID NO:15), marker locus M9 (SEQ ID NO:16), marker locus M10 (SEQ ID NO:17), marker locus M11 (SEQ ID NO:18), marker locus M12 (SEQ ID NO:19), marker locus M13 (SEQ ID NO:2), marker locus M14 (SEQ ID NO:3), marker locus M15 (SEQ ID NO:4), marker locus M16 (SEQ ID NO:5), marker locus M17 (SEQ ID NO:6), marker locus M18 (SEQ ID NO:7), marker locus M19 (SEQ ID NO:8), and marker locus M20 (SEQ ID NO:9).
13. The method of any one of embodiments 1 to 12, wherein screening said population comprises PCR, single strand conformational polymorphism analysis, denaturing gradient gel electrophoresis, cleavage fragment length polymorphism analysis, TAQMAN assay, and/or DNA sequencing.
14. The method of any one of embodiments 1 to 13, wherein selecting one or more plants comprises:
   (a) detecting a marker locus within or genetically linked to a chromosomal segment flanked in the genome of said plant by marker locus M1 (SEQ ID NO: 11) and marker locus M2 (SEQ ID NO:20) on chromosome 2;
   (b) detecting a marker locus within or genetically linked to a chromosomal segment flanked in the genome of said plant by marker locus M3 (SEQ ID NO:1) and marker locus M4 (SEQ ID NO: 10) on chromosome 6; or
   (c) detecting at least one polymorphism at a locus selected from the group consisting of marker locus marker locus M5 (SEQ ID NO:12), marker locus M6 (SEQ ID NO:13), marker locus M7 (SEQ ID NO: 14), marker locus M8 (SEQ ID NO:15), marker locus M9 (SEQ ID NO:16), marker locus M10 (SEQ ID NO:17), marker locus M11 (SEQ ID NO:18), marker locus M12 (SEQ ID NO:19), marker locus M13 (SEQ ID NO:2), marker locus M14 (SEQ ID NO:3), marker locus M15 (SEQ ID NO:4), marker locus M16 (SEQ ID NO:5), marker locus M17 (SEQ ID NO:6), marker locus M18 (SEQ ID NO:7), marker locus M19 (SEQ ID NO:8), and marker locus M20 (SEQ ID NO: 9).
15. A cucumber plant produced by the method of one of embodiments 1 to 14, wherein said plant comprises said CMV resistance allele within a chromosomal segment flanked in the genome of said plant by marker locus M1 (SEQ ID NO: 11) and marker locus M2 (SEQ ID NO:20) on chromosome 2, and said CMV resistance allele within a chromosomal segment flanked in the genome of said plant by marker locus M3 (SEQ ID NO:1) and marker locus M4 (SEQ ID NO: 10) on chromosome 6, and wherein the plant further comprises a CMV resistance allele within a chromosomal segment flanked in the genome of said plant by:
   marker locus M21 (SEQ ID NO:29) and marker locus M22 (SEQ ID NO:30) on chromosome 5; or
   marker locus KT316424 and marker locus KT316425 on chromosome 5.
15. A method of producing a cucumber plant exhibiting resistance to *Cucumber mosaic virus* (CMV), comprising introgressing into a plant a *Cucumber mosaic virus* resistance allele within a chromosomal segment flanked in the genome of said plant by marker locus M1 (SEQ ID NO:11) and marker locus M2 (SEQ ID NO:20) on chromosome 2; or marker locus M3 (SEQ ID NO:1) and marker locus M4 (SEQ ID NO:10) on chromosome 6; wherein said introgressed CMV resistance allele confers to said plant increased resistance to CMV compared to a plant not comprising said allele.
16. The method of embodiment 15, wherein said introgressing comprises:
   a) crossing a cucumber plant comprising said chromosomal segment with itself or with a second cucumber plant to produce one or more progeny plants; and
   b) selecting a progeny plant comprising said chromosomal segment.
17. The method of one of embodiments 15 or 16, wherein said cucumber plant comprises said chromosomal segment on chromosome 2, and wherein a representative sample of seed comprising said chromosomal segment has been deposited under ATCC Accession No. PTA-122638.
18. The method of one of embodiments 15 to 17, wherein said cucumber plant comprises said chromosomal segment on chromosome 6, and wherein a representative sample of seed comprising said chromosomal segment has been deposited under ATCC Accession No. PTA-122638.
19. The method of one of embodiments 15 to 18, wherein the *Cucumber mosaic virus* resistance allele is further defined as:
   a) located within a chromosomal segment flanked in the genome of said plant by marker locus M24 (SEQ ID NO:32) and marker locus M25 (SEQ ID NO:33) on chromosome 2; or
   b) located within a chromosomal segment flanked in the genome of said plant by marker locus M26 (SEQ ID NO:34) and marker locus M27 (SEQ ID NO:35) on chromosome 6.
20. The method of one of embodiments 15 to 19, wherein said chromosomal segment comprises a marker locus on chromosome 2 selected from the group consisting of marker locus M5 (SEQ ID NO: 12), marker locus M6 (SEQ ID NO: 13), marker locus M7 (SEQ ID NO:14), marker locus M8 (SEQ ID NO:15), marker locus M9 (SEQ ID NO:16), marker locus M10 (SEQ ID NO:17), marker locus M11 (SEQ ID NO:18), and marker locus M12 (SEQ ID NO:19).
21. The method of one of embodiments 15 to 20, wherein said chromosomal segment comprises a marker locus on chromosome 6 selected from the group consisting of marker locus M13 (SEQ ID NO:2), marker locus M14 (SEQ ID NO:3), marker locus M15 (SEQ ID NO:4), marker locus M16 (SEQ ID NO:5), marker locus M17 (SEQ ID NO:6), marker locus M18 (SEQ ID NO:7), marker locus M19 (SEQ ID NO:8), and marker locus M20 (SEQ ID NO: 9).
22. The method of one of embodiments 15 to 21, wherein said method further comprises introgressing into the plant a CMV resistance allele within a chromosomal segment flanked in the genome of said plant by:
   marker locus M21 (SEQ ID NO:29) and marker locus M22 (SEQ ID NO:30) on chromosome 5;
   marker locus KT316424 and marker locus KT316425 on chromosome 5; or
   marker locus SSR9-56 and marker locus SSR11-177 on chromosome 6.
23. The method of one of embodiments 15 to 22, wherein said method comprises introgressing into the plant a CMV resistance allele within a chromosomal segment flanked in the genome of said plant by marker locus M1 (SEQ ID NO: 11) and marker locus M2 (SEQ ID NO:20) on chromosome 2 and a CMV resistance allele within a chromosomal segment flanked in the genome of said plant by marker locus M3 (SEQ ID NO:1) and marker locus M4 (SEQ ID NO: 10) on chromosome 6.
24. The method of one of embodiments 15 23, wherein said introgressing comprises backcrossing, marker-assisted selection, or assaying for said CMV resistance.
25. A cucumber plant produced by the method of one of embodiments 15 to 24, wherein said plant comprises said CMV resistance allele within a chromosomal segment flanked in the genome of said plant by marker locus M1 (SEQ ID NO: 11) and marker locus M2 (SEQ ID NO:20) on chromosome 2, and said CMV resistance allele within a chromosomal segment flanked in the genome of said plant by marker locus M3 (SEQ ID NO: 1) and marker locus M4 (SEQ ID NO: 10) on chromosome 6, and wherein the plant further comprises a CMV resistance allele within a chromosomal segment flanked in the genome of said plant by:
   marker locus M21 (SEQ ID NO:29) and marker locus M22 (SEQ ID NO:30) on chromosome 5; or
   marker locus KT316424 and marker locus KT316425 on chromosome 5.
26. A method for selecting a cucumber plant with increased resistance to *Cucumber mosaic virus* (CMV) comprising:
   (a) crossing a cucumber plant comprising a CMV resistance allele with a second cucumber plant to produce a population of progeny plants; and
   (b) selecting a progeny plant comprising said CMV resistance allele;
   wherein selecting said progeny plant comprises detecting a marker locus within or genetically linked to a chromosomal segment flanked in the genome of said plant by:
   marker locus M1 (SEQ ID NO:11) and marker locus M2 (SEQ ID NO:20) on chromosome 2; or
   marker locus M3 (SEQ ID NO:1) and marker locus M4 (SEQ ID NO:10) on chromosome 6.
27. The method of embodiment 26, wherein selecting said progeny plant is further defined as detecting a marker locus within or genetically linked to a chromosomal segment flanked in the genome of said plant by:
   a) marker locus M24 (SEQ ID NO:32) and marker locus M25 (SEQ ID NO:33) on chromosome 2; or
   b) marker locus M26 (SEQ ID NO:34) and marker locus M27 (SEQ ID NO:35) on chromosome 6.
28. The method of one of embodiments 26 or 27, wherein selecting a progeny plant comprises detecting nucleic acids comprising marker locus M5 (SEQ ID NO:12), marker locus M6 (SEQ ID NO:13), marker locus M7 (SEQ ID NO: 14), marker locus M8 (SEQ ID NO: 15), marker locus M9 (SEQ ID NO:16), marker locus M10 (SEQ ID NO: 17), marker locus M11 (SEQ ID NO: 18), marker locus M12 (SEQ ID NO: 19), marker locus M13 (SEQ ID NO:2), marker locus M14 (SEQ ID NO:3), marker locus M15 (SEQ ID NO:4), marker locus M16 (SEQ ID NO:5), marker locus M17 (SEQ ID NO:6), marker locus M18 (SEQ ID NO:7), marker locus M19 (SEQ ID NO:8), or marker locus M20 (SEQ ID NO:9).
29. The method of one of embodiments 26 to 28, wherein the progeny plant is an F₂-F₆ progeny plant.
30. The method of one of embodiments 26 to 29, wherein producing said progeny plant comprises backcrossing.
31. A method of selecting a cucumber plant exhibiting resistance to *Cucumber mosaic virus* (CMV), comprising:
   a) obtaining a population of progeny plants having a parent comprising resistance to CMV;
   b) screening said population with at least one nucleic acid marker to detect a polymorphism genetically linked to CMV resistance; and
   c) selecting from said population one or more progeny plants comprising a haplotype associated with CMV resistance, wherein the haplotype comprises a CMV resistance allele flanked in the genome of said plant by:
      marker locus M1 (SEQ ID NO:11) and marker locus M2 (SEQ ID NO:20) on chromosome 2; or
      marker locus M3 (SEQ ID NO:1) and marker locus M4 (SEQ ID NO:10) on chromosome 6.
32. The method of embodiment 31, wherein selecting said progeny plant comprises:
   (a) detecting a marker locus within or genetically linked to a chromosomal segment flanked in the genome of said plant by marker locus M1 (SEQ ID NO: 11) and marker locus M2 (SEQ ID NO:20) on chromosome 2;
   (b) detecting a marker locus within or genetically linked to a chromosomal segment flanked in the genome of said plant by marker locus M3 (SEQ ID NO:1) and marker locus M4 (SEQ ID NO: 10) on chromosome 6; or
   (c) detecting at least one polymorphism at a locus selected from the group consisting of marker locus marker locus M5 (SEQ ID NO:12), marker locus M6 (SEQ ID NO:13), marker locus M7 (SEQ ID NO: 14), marker locus M8 (SEQ ID NO:15), marker locus M9 (SEQ ID NO:16), marker locus M10 (SEQ ID NO:17), marker locus M11 (SEQ ID NO:18), marker locus M12 (SEQ ID NO:19), marker locus M13 (SEQ ID NO:2), marker locus M14 (SEQ ID NO:3), marker locus M15 (SEQ ID NO:4), marker locus M16 (SEQ ID NO:5), marker locus M17 (SEQ ID NO:6), marker locus M18 (SEQ ID NO:7), marker locus M19 (SEQ ID NO:8), and marker locus M20 (SEQ ID NO:9).
33. The method of one of embodiments 31 or 32, wherein said progeny plant is an F₂-F₆ progeny plant.
34. The method of one of embodiments 31 to 33, wherein producing said progeny plant comprises backcrossing.
35. The method of one of embodiments 31 to 34, wherein screening said population comprises PCR, single strand conformational polymorphism analysis, denaturing gradient gel electrophoresis, cleavage fragment length polymorphism analysis, TAQMAN assay, and/or DNA sequencing.
36. The method of one of embodiments 31 to 35, wherein the CMV resistance cucumber plant comprises a CMV resistance allele on chromosome 2, and wherein a representative sample of seed comprising said allele has been deposited under ATCC Accession No. PTA-122638.
37. The method of one of embodiments 31 to 36, wherein the CMV resistance cucumber plant comprises a CMV resistance allele on chromosome 6, and wherein a representative sample of seed comprising said allele has been deposited under ATCC Accession No. PTA-122638.
38. A method of selecting a cucumber plant exhibiting resistance to *Cucumber mosaic virus* (CMV), comprising:
   a) screening one or more plants with at least one nucleic acid marker to detect a polymorphism genetically linked to CMV resistance; and
   b) selecting one or more plants comprising a haplotype associated with CMV resistance, wherein the haplotype comprises a CMV resistance allele flanked in the genome of said plant by:
      marker locus M1 (SEQ ID NO:11) and marker locus M2 (SEQ ID NO:20) on chromosome 2; or
      marker locus M3 (SEQ ID NO:1) and marker locus M4 (SEQ ID NO:10) on chromosome 6.
39. The method of embodiment 38, wherein the CMV resistance allele is further defined as:
   a) located within a chromosomal segment flanked in the genome of said plant by marker locus M24 (SEQ ID NO:32) and marker locus M25 (SEQ ID NO:33) on chromosome 2; or
   b) located within a chromosomal segment flanked in the genome of said plant by marker locus M26 (SEQ ID NO:34) and marker locus M27 (SEQ ID NO:35) on chromosome 6.
40. The method of one of embodiments 38 or 39, wherein selecting one or more plants comprises:
   (a) detecting a marker locus within or genetically linked to a chromosomal segment flanked in the genome of said plant by marker locus M1 (SEQ ID NO: 11) and marker locus M2 (SEQ ID NO:20) on chromosome 2;
   (b) detecting a marker locus within or genetically linked to a chromosomal segment flanked in the genome of said plant by marker locus M3 (SEQ ID NO:1) and marker locus M4 (SEQ ID NO: 10) on chromosome 6; or
   (c) detecting at least one polymorphism at a locus selected from the group consisting of marker locus marker locus M5 (SEQ ID NO:12), marker locus M6 (SEQ ID NO:13), marker locus M7 (SEQ ID NO: 14), marker locus M8 (SEQ ID NO:15), marker locus M9 (SEQ ID NO: 16), marker locus M10 (SEQ ID NO: 17), marker locus M11 (SEQ ID NO:18), marker locus M12 (SEQ ID NO:19), marker locus M13 (SEQ ID NO:2), marker locus M14 (SEQ ID NO:3), marker locus M15 (SEQ ID NO:4), marker locus M16 (SEQ ID NO:5), marker locus M17 (SEQ ID NO:6), marker locus M18 (SEQ ID NO:7), marker locus M19 (SEQ ID NO:8), and marker locus M20 (SEQ ID NO:9).
41. The method of one of embodiments 38 to 40, wherein screening one or more plants comprises PCR, single strand conformational polymorphism analysis, denaturing gradient gel electrophoresis, cleavage fragment length polymorphism analysis, TAQMAN assay, and/or DNA sequencing.
42. A method for identifying a cucumber plant comprising a *Cucumber mosaic virus* (CMV) resistance allele:
   (a) obtaining nucleic acids from at least a first cucumber plant; and
   (b) identifying in said nucleic acids the presence of at least a first genetic marker indicative of the presence of a chromosomal segment flanked in the genome of said plant by:
      marker locus M1 (SEQ ID NO:11) and marker locus M2 (SEQ ID NO:20) on chromosome 2; or
      marker locus M3 (SEQ ID NO:1) and marker locus M4 (SEQ ID NO:10) on chromosome 6
      wherein said CMV resistance allele confers to said plant increased resistance to CMV compared to a plant not comprising said allele.
43. The method of embodiment 42, wherein said identifying comprises detecting a marker genetically linked to:
   (a) marker locus M5 (SEQ ID NO:12), marker locus M6 (SEQ ID NO:13), marker locus M7 (SEQ ID NO:14), marker locus M8 (SEQ ID NO:15), marker locus M9 (SEQ ID NO:16), marker locus M10 (SEQ ID NO:17), marker locus M11 (SEQ ID NO:18), and marker locus M12 (SEQ ID NO:19); or
   (b) marker locus M13 (SEQ ID NO:2), marker locus M14 (SEQ ID NO:3), marker locus M15 (SEQ ID NO:4), marker locus M16 (SEQ ID NO:5), marker locus M17 (SEQ ID NO:6), marker locus M18 (SEQ ID NO:7), marker locus M19 (SEQ ID NO:8), and marker locus M20 (SEQ ID NO:9).

## Claims

1. A method of selecting a cucumber plant exhibiting resistance to *Cucumber mosaic virus* (CMV), comprising:
a) screening one or more plants with at least one nucleic acid marker to detect a polymorphism genetically linked to CMV resistance; and
b) selecting one or more plants comprising a haplotype associated with CMV resistance, wherein the haplotype comprises a CMV resistance allele flanked in the genome of said plant by:
marker locus M1 (SEQ ID NO:11) and marker locus M2 (SEQ ID NO:20) on chromosome 2; or
marker locus M3 (SEQ ID NO:1) and marker locus M4 (SEQ ID NO:10) on chromosome 6.

2. A method for identifying a cucumber plant comprising a *Cucumber mosaic virus* (CMV) resistance allele:
(a) obtaining nucleic acids from at least a first cucumber plant; and
(b) identifying in said nucleic acids the presence of at least a first genetic marker indicative of the presence of a chromosomal segment flanked in the genome of said plant by:
marker locus M1 (SEQ ID NO:11) and marker locus M2 (SEQ ID NO:20) on chromosome 2; or
marker locus M3 (SEQ ID NO:1) and marker locus M4 (SEQ ID NO:10) on chromosome 6
wherein said CMV resistance allele confers to said plant increased resistance to CMV compared to a plant not comprising said allele.

3. A method for selecting a cucumber plant with increased resistance to *Cucumber mosaic virus* (CMV) comprising:
selecting a plant from a population of plants, at least some of comprise a CMV resistance,
wherein selecting comprises detecting a marker locus within or genetically linked to a chromosomal segment flanked in the genome of said plant by:
marker locus M1 (SEQ ID NO:11) and marker locus M2 (SEQ ID NO:20) on chromosome 2; or
marker locus M3 (SEQ ID NO:1) and marker locus M4 (SEQ ID NO:10) on chromosome 6.

4. A method of selecting a cucumber plant exhibiting resistance to *Cucumber mosaic virus* (CMV), comprising:
a) obtaining a population of plants having a parent comprising resistance to CMV;
b) screening said population with at least one nucleic acid marker to detect a polymorphism genetically linked to CMV resistance; and
c) selecting from said population one or more plants comprising a haplotype associated with CMV resistance, wherein the haplotype comprises a CMV resistance allele flanked in the genome of said plant by:
marker locus M1 (SEQ ID NO:11) and marker locus M2 (SEQ ID NO:20) on chromosome 2; or
marker locus M3 (SEQ ID NO:1) and marker locus M4 (SEQ ID NO:10) on chromosome 6.

5. A method of producing a cucumber plant exhibiting resistance to *Cucumber mosaic virus* (CMV), comprising introgressing into a plant a *Cucumber mosaic virus* resistance allele within a chromosomal segment flanked in the genome of said plant by marker locus M1 (SEQ ID NO:11) and marker locus M2 (SEQ ID NO:20) on chromosome 2; or marker locus M3 (SEQ ID NO:1) and marker locus M4 (SEQ ID NO:10) on chromosome 6; wherein said introgressed CMV resistance allele confers to said plant increased resistance to CMV compared to a plant not comprising said allele.

6. The method of any one of claims 1 to 5, wherein a representative sample of seed of a cucumber plant comprising resistance to CMV has been deposited under ATCC Accession No. PTA-122638.

7. The method of any one of claims 1 to 6, wherein the *Cucumber mosaic virus* resistance allele is further defined as:
a) located within a chromosomal segment flanked in the genome of said plant by marker locus M24 (SEQ ID NO:32) and marker locus M25 (SEQ ID NO:33) on chromosome 2; or
b) located within a chromosomal segment flanked in the genome of said plant by marker locus M26 (SEQ ID NO:34) and marker locus M27 (SEQ ID NO:35) on chromosome 6.

8. The method of any one of claims 1 to 7, wherein said chromosomal segment comprises a marker locus on chromosome 2 selected from the group consisting of marker locus M5 (SEQ ID NO:12), marker locus M6 (SEQ ID NO:13), marker locus M7 (SEQ ID NO:14), marker locus M8 (SEQ ID NO:15), marker locus M9 (SEQ ID NO:16), marker locus M10 (SEQ ID NO:17), marker locus M11 (SEQ ID NO:18), and marker locus M12 (SEQ ID NO:19).

9. The method of any one of claims 1 to 8, wherein said chromosomal segment comprises a marker locus on chromosome 6 selected from the group consisting of marker locus M13 (SEQ ID NO:2), marker locus M14 (SEQ ID NO:3), marker locus M15 (SEQ ID NO:4), marker locus M16 (SEQ ID NO:5), marker locus M17 (SEQ ID NO:6), marker locus M18 (SEQ ID NO:7), marker locus M19 (SEQ ID NO:8), and marker locus M20 (SEQ ID NO:9).

10. The method of claim 9, wherein selecting said progeny plant is further defined as detecting a marker locus within or genetically linked to a chromosomal segment flanked in the genome of said plant by:
a) marker locus M24 (SEQ ID NO:32) and marker locus M25 (SEQ ID NO:33) on chromosome 2; or
b) marker locus M26 (SEQ ID NO:34) and marker locus M27 (SEQ ID NO:35) on chromosome 6.

11. The method of any one of claims 1 to 10, wherein selecting a progeny plant comprises detecting nucleic acids comprising marker locus M5 (SEQ ID NO:12), marker locus M6 (SEQ ID NO:13), marker locus M7 (SEQ ID NO:14), marker locus M8 (SEQ ID NO:15), marker locus M9 (SEQ ID NO:16), marker locus M10 (SEQ ID NO:17), marker locus M11 (SEQ ID NO:18), marker locus M12 (SEQ ID NO:19), marker locus M13 (SEQ ID NO:2), marker locus M14 (SEQ ID NO:3), marker locus M15 (SEQ ID NO:4), marker locus M16 (SEQ ID NO:5), marker locus M17 (SEQ ID NO:6), marker locus M18 (SEQ ID NO:7), marker locus M19 (SEQ ID NO:8), or marker locus M20 (SEQ ID NO:9).

12. The method of any one of claims 1 to 11, wherein selecting said plant comprises:
(a) detecting a marker locus within or genetically linked to a chromosomal segment flanked in the genome of said plant by marker locus M1 (SEQ ID NO:11) and marker locus M2 (SEQ ID NO:20) on chromosome 2;
(b) detecting a marker locus within or genetically linked to a chromosomal segment flanked in the genome of said plant by marker locus M3 (SEQ ID NO:1) and marker locus M4 (SEQ ID NO:10) on chromosome 6; or
(c) detecting at least one polymorphism at a locus selected from the group consisting of marker locus marker locus M5 (SEQ ID NO:12), marker locus M6 (SEQ ID NO:13), marker locus M7 (SEQ ID NO:14), marker locus M8 (SEQ ID NO:15), marker locus M9 (SEQ ID NO:16), marker locus M10 (SEQ ID NO:17), marker locus M11 (SEQ ID NO:18), marker locus M12 (SEQ ID NO:19), marker locus M13 (SEQ ID NO:2), marker locus M14 (SEQ ID NO:3), marker locus M15 (SEQ ID NO:4), marker locus M16 (SEQ ID NO:5), marker locus M17 (SEQ ID NO:6), marker locus M18 (SEQ ID NO:7), marker locus M19 (SEQ ID NO:8), and marker locus M20 (SEQ ID NO:9).

13. The method of any one of claims 1 to 12, wherein screening said population comprises PCR, single strand conformational polymorphism analysis, denaturing gradient gel electrophoresis, cleavage fragment length polymorphism analysis, TAQMAN assay, and/or DNA sequencing.

14. The method of any one of claims 1 to 13, wherein selecting one or more plants comprises:
(a) detecting a marker locus within or genetically linked to a chromosomal segment flanked in the genome of said plant by marker locus M1 (SEQ ID NO:11) and marker locus M2 (SEQ ID NO:20) on chromosome 2;
(b) detecting a marker locus within or genetically linked to a chromosomal segment flanked in the genome of said plant by marker locus M3 (SEQ ID NO:1) and marker locus M4 (SEQ ID NO:10) on chromosome 6; or
(c) detecting at least one polymorphism at a locus selected from the group consisting of marker locus marker locus M5 (SEQ ID NO:12), marker locus M6 (SEQ ID NO:13), marker locus M7 (SEQ ID NO:14), marker locus M8 (SEQ ID NO:15), marker locus M9 (SEQ ID NO:16), marker locus M10 (SEQ ID NO:17), marker locus M11 (SEQ ID NO:18), marker locus M12 (SEQ ID NO:19), marker locus M13 (SEQ ID NO:2), marker locus M14 (SEQ ID NO:3), marker locus M15 (SEQ ID NO:4), marker locus M16 (SEQ ID NO:5), marker locus M17 (SEQ ID NO:6), marker locus M18 (SEQ ID NO:7), marker locus M19 (SEQ ID NO:8), and marker locus M20 (SEQ ID NO:9).

15. A cucumber plant produced by the method of claim 1, wherein said plant comprises said CMV resistance allele within a chromosomal segment flanked in the genome of said plant by marker locus M1 (SEQ ID NO:11) and marker locus M2 (SEQ ID NO:20) on chromosome 2, and said CMV resistance allele within a chromosomal segment flanked in the genome of said plant by marker locus M3 (SEQ ID NO:1) and marker locus M4 (SEQ ID NO:10) on chromosome 6, and wherein the plant further comprises a CMV resistance allele within a chromosomal segment flanked in the genome of said plant by:
marker locus M21 (SEQ ID NO:29) and marker locus M22 (SEQ ID NO:30) on chromosome 5; or
marker locus KT316424 and marker locus KT316425 on chromosome 5.
